# EUROPEAN PATENT APPLICATION

(11) **EP 4 458 958 A1**
(43) Date of publication of application: **06.11.2024**
(21) Application number: 21969852.9
(22) Date of filing: 31.12.2021
(51) Int. Cl.: C12N 9/02, C12Q 1/66

(54) **MUTANT AND USES THEREOF**

(71) Applicant: BGI Shenzhen, Shenzhen, Guangdong 518083 (CN)
(72) Inventor: ZHANG, Wei, Shenzhen, Guangdong 518083 (CN); DONG, Yuliang, Shenzhen, Guangdong 518083 (CN); LI, Jing, Shenzhen, Guangdong 518083 (CN); ZHENG, Yue, Shenzhen, Guangdong 518083 (CN); ZHANG, Wenwei, Shenzhen, Guangdong 518083 (CN)
(74) Representative: Schwarz & Partner Patentanwälte GmbH
(86) International application number: PCT/CN2021/144051
(87) International publication number: WO 2023/123509

(57) **Abstract**

A mutant and the uses thereof. Compared with the amino acid sequence shown in SEQ ID NO: 2, the mutant has at least one of the following mutation sites: the 24th, 26th, 27th, 29th, 30th, 31st, 32nd, 33rd, 36th, 37th, 40th, 66th, 79th, 84th, 88th, 102nd, 103rd, 104th, 110th, 123rd, 124th, 138th, 152nd, 163rd, 167th, 170th, 174th, 175th, 178th, 182nd and 183rd sites.

## Description

### Priority

None.

### FIELD

The present disclosure relates to the technical field of biology, specifically, relates to mutants and use thereof.

### BACKGROUND

Luciferase, refers to a generic name of a class of enzymes that catalyze oxidation of luciferin or fatty aldehyde for luminescence in organisms, and is usually found in lower animals. Commonly used luciferase at present includes firefly luciferase, renilla luciferase, Gaussia luciferase, etc. The firefly luciferase needs the assistance of ATP and Mg²⁺ for luminescence. Although independent of auxiliary factors such as ATP and Mg²⁺, renilla luciferase catalyzes so weak luminescence intensity that more sensitive detection is needed. As independent of auxiliary factors such as ATP and Mg²⁺ and catalyzing luminescence intensity 100 times higher than that of renilla luciferase, Gaussia luciferase well makes up for the shortcomings of firefly luciferase and ranilla luciferase.

With this self-luminescence property, luciferase is often used in the fields of live cell detection, protein-protein interaction, protein localization, small interfering RNA silencing and high-throughput drug screening. Luciferase may be used to detect the presence or absence of chemical pollutants in the field of biological monitoring technology. Additionally, it also has broader application prospects in fields of immune detection, biochemical diagnosis, etc. However, in order to detect the expression intensity and transcriptional regulation of foreign genes under different promoters, as reporter genes, a variety of luciferase with similar self-luminescence brightness and different catalytic substrates are required to be combined for use. Therefore, there is in need of developing more luciferase that catalyze different substrates, emit more intensive light, and are detectable without sensitivity detection technology.

### SUMMARY

The present disclosure is based on the following discovery of the inventor: a Gaussia luciferase mutant with a luminescence intensity for a substrate coelenterazine (CTZ) at most 6 times as bright as that of the wild type, an increased catalytic activity on a substrate fluoro-coelenterazine (f-CTZ) by 25.7 times, and an increased catalytic activity on a substrate coelenterazine derivative ZS2 by 22.7 times, is obtained with directed protein evolution for Gaussia luciferase by the inventors. This luciferase may be expressed in prokaryote and purified with a simple process, and thus could be produced in large-scale; and the luciferase is easily detectable in view of a simple detecting method for a luciferase activity thereof. Therefore, the luciferase according to embodiments of the present disclosure has broad application prospects in basic scientific research, biological monitoring, biochemical diagnosis and other fields.

Thus, in a first aspect, the present disclosure provides in embodiments a mutant. According to the embodiment of the present disclosure, the mutant includes at least one mutation, compared with an amino acid sequence as shown in SEQ ID NO: 3 occurred at a position(s) 24, 26, 27, 29, 30, 31, 32, 33, 36, 37, 40, 66, 79, 84, 88, 102, 103, 104, 110, 123, 124, 138, 152, 163, 167, 170, 174, 175, 178, 182, and 183 thereof. The mutant according to the embodiment of the present disclosure has a strong catalytic activity on substrates such as coelenterazine, fluoro-coelenterazine and coelenterazine derivatives, and has a wider substrate spectrum or higher substrate selectivity specificity and significantly enhanced luminescence brightness therein, compared with the existing Gaussia luciferase. Therefore, the mutant may be applied in the fields involving with luminescent detection, such as basic scientific research, biological detection, immune detection, biochemical detection, or diagnosis, and has broad application prospects.

In a second aspect, the present disclosure provides in embodiments a nucleic acid molecule. According to the embodiment of the present disclosure, the nucleic acid molecule encodes the mutant as described in the embodiments of the first aspect. According to the embodiment of the present disclosure, the mutant encoded by the nucleic acid molecule has a strong catalytic activity on substrates such as coelenterazine, fluoro-coelenterazine and coelenterazine derivatives, and has a wider substrate spectrum or higher substrate selectivity specificity and significantly enhanced luminescence brightness therein, compared with the existing Gaussia luciferase. Therefore, the mutant protein may be applied in the fields involving with luminescent detection, such as basic scientific research, biological detection, immune detection, biochemical detection, or diagnosis, and has broad application prospects.

In a third aspect, the present disclosure provides in embodiments an expression vector. According to the embodiment of the present disclosure, the nucleic acid molecule as described in the embodiments of the second aspect is included. The expression vector may include an optional control sequence operably connected with the nucleic acid molecule, wherein the control sequence refers to one or more control sequences guiding an expression of the nucleic acid molecule in a host. The expression vector provided in the embodiment of the present disclosure may efficiently express a protein in suitable host cells, and the obtained protein has a strong catalytic activity on substrates such as coelenterazine, fluoro-coelenterazine and coelenterazine derivatives, and has a wider substrate spectrum or higher substrate selectivity specificity and significantly enhanced luminescence brightness therein, compared with the existing Gaussia luciferase. Therefore, the mutant protein may be applied in the fields involving with luminescent detection, such as basic scientific research, biological detection, immune detection, biochemical detection, or diagnosis, and has broad application prospects.

In a fourth aspect, the present disclosure provides in embodiments a recombinant cell. According to the embodiment of the present disclosure, the recombinant cell carries the nucleic acid molecule as described in the embodiment of the second aspect, the expression vector as described in the embodiment of the third aspect, or the mutant as described in the embodiment of the first aspect. The recombinant cells are obtained by transfecting or transforming the expression vector. According to the embodiment of the present disclosure, the recombinant cell may efficiently express the above mutant under appropriate conditions, and the mutant has a strong catalytic activity on substrates such as coelenterazine, fluoro-coelenterazine and coelenterazine derivatives, and has a wider substrate spectrum or higher substrate selectivity specificity and significantly enhanced luminescence brightness therein, compared with the existing Gaussia luciferase. Therefore, the mutant may be applied in the fields involving with luminescent detection, such as basic scientific research, biological detection, immune detection, biochemical detection, or diagnosis, and has broad application prospects.

In a fifth aspect, the present disclosure provides in embodiments a method for detecting a nucleic acid. According to the embodiment of the present disclosure, the method includes: i) exposing an expression vector to an environment suitable for protein expression, wherein the expression vector contains the nucleic acid to be detected and a nucleic acid molecule as described above, the nucleic acid to be detected is operably connected to and expressed together with the nucleic acid molecule as described above; ii) introducing a substrate for Gaussia luciferase or analog thereof into the environment suitable for protein expression; and iii) determining an expression of the nucleic acid to be detected based on a fluorescence intensity change in the environment suitable for protein expression. Those skilled in the art would learn that the expression of the nucleic acid may produce an RNA or protein. According to the specific embodiment of the present disclosure, the mutant encoded by the nucleic acid molecule has a strong catalytic activity on substrates such as coelenterazine, fluoro-coelenterazine and coelenterazine derivatives, and has a wider substrate spectrum or higher substrate selectivity specificity and significantly enhanced luminescence brightness therein, compared with the existing Gaussia luciferase. Therefore, the method may be used to detect a nucleic acid expressing to an RNA or protein for example, an expression level of an RNA or protein, or a location or tracing of a protein, with significantly higher accuracy and sensitivity than using the existing Gaussia luciferase, thereby obtaining more accurate results.

In a sixth aspect, the present disclosure provides in embodiments a method for preparing a mutant. According to the embodiment of the present disclosure, the method includes: i) constructing an expression vector as described in the embodiments of the third aspect; ii) introducing the expression vector into a host cell to obtain the mutant. According to the embodiment of the present disclosure, the mutant may be obtained simply and efficiently through the method, and the mutant has a strong catalytic activity on substrates such as coelenterazine, fluoro-coelenterazine and coelenterazine derivatives, and has a wider substrate spectrum or higher substrate selectivity specificity and significantly enhanced luminescence brightness therein, compared with the existing Gaussia luciferase. Therefore, the protein mutant may be applied in the fields involving with luminescent detection, such as basic scientific research, biological detection, immune detection, biochemical detection, or diagnosis, and has broad application prospects.

In a seventh aspect, the present disclosure provides in embodiments use of a mutant as described in the embodiments of the first aspect in the preparation of a kit. According to the embodiment of the present disclosure, the kit is used to detecting a content of analyte, wherein a specific recognition protein of the analyte allows to form a complex with the mutant. Utilizing properties of the mutant reacting with the substrate via the binding therebetween, the mutant may be used as a signal protein for detecting the analyte. For example, by coupling or fusing the mutant with a protein capable of specifically recognizing the analyte by means of chemical coupling or fusion protein forming, when the mutant, the analyte, and the substrate are present in the same system, the protein specifically recognizing the analyte would bind to the analyte and the mutant thereamong would catalyze the substrate, to perform the self-luminescence. The intensity of the bioluminescence released during the catalytic process of the mutant on the substrate may be measured with a microplate reader for chemiluminescence, and this intensity of the bioluminescence reflects the activity of the mutant, and the content of the analyte may be determined by such an activity level. It should be noted that before the binding of the substrate with the mutant, non-specific binding proteins in the system need to be removed, so as to eliminate the interference of other factors on the results. Therefore, the mutant may be used to prepare the kit to accurately detect a content of analyte.

In an eighth aspect, the present disclosure provides in embodiments a kit for detecting a content of analyte. According to embodiments of the present disclosure, the kit includes a mutant as described in the embodiments of the first aspect, wherein a specific recognition protein of the analyte allows to form a complex with the mutant. Utilizing properties of the mutant reacting with the substrate via the binding therebetween, the mutant may be used as a signal protein for detecting the analyte. For example, a protein capable of specifically recognizing the analyte, by means of chemical coupling or fusion protein forming, may be coupled with or fused on the mutant, and the protein specifically recognizing the analyte would bind to the analyte, and the mutant thereamong would catalyze the substrate to present the self-luminescence. The intensity of the bioluminescence released during the catalytic process of the mutant on the substrate may be measured with a microplate reader for chemiluminescence, and this intensity of the bioluminescence reflects the activity of the mutant, and the content of the analyte may be determined by such an activity level. Thus, the kit including the mutant may be used to detect a content of analyte accurately.

In a ninth aspect, the present disclosure provides in embodiments a method for detecting a content of analyte, including: i) rending a specific recognition protein of analyte and a mutant as described above to form a complex, ii) contacting the analyte with the complex, iii) introducing a substrate for Gaussia luciferase or analog thereof into the system, and iv) determining the content of the analyte based on a fluorescence intensity change caused by the mutant before and after introducing the substrate for Gaussia luciferase or analog thereof. Utilizing properties of the mutant reacting with the substrate via the binding therebetween, a protein specifically recognizing the analyte may be coupled with or fused to the mutant by means of chemical coupling or fusion protein forming, which may specifically bind with the analyte, and the mutant therein has the ability to catalyze the luminescence of the substrate for Gaussia luciferase or analog thereof. When the fused or coupled protein is combined with the analyte, with removing non-specific binding proteins in the system, the substrate for Gaussia luciferase or analog thereof is introduced into the system, to measure, by a microplate reader for chemiluminescent, the bioluminescence released during the luminescence process of the substrate catalyzed by the mutant. As the bioluminescence measured reflects the combination of the mutant and the substrate, the content of the analyte may be determined by such an intensity. In addition, the mutant has a strong catalytic activity on substrates such as coelenterazine, fluoro-coelenterazine and coelenterazine derivatives, and has a wider substrate spectrum or higher substrate selectivity specificity and significantly enhanced luminescence brightness therein compared with the existing Gaussia luciferase, and the method according to the embodiment of the present disclosure can therefore detect a content of analyte more accurately.

In a tenth aspect, the present disclosure provides in embodiments a method for screening a substrate for Gaussia luciferase. According to the embodiment of the present disclosure, the method includes: i) contacting a mutant in the embodiments of the first aspect with a substrate to be screened, and ii) determining whether the substrate to be screened is a target substrate or not based on whether a mixture in step i) emitting a chemical light signal or not. Utilizing properties of the mutant reacting with the substrate via the binding therebetween, the substrate of interest to be screened is contacted with the mutant, which will release bioluminescence during the process of catalyzing the substrate to be screened. By determining whether the mutant catalyzes the substrate to be screened to emit chemical luminescence or not with a microplate reader chemiluminescence, the substrate to be screened may be determined whether as the target substrate or not. Therefore, the method according to the embodiment of the present disclosure can accurately screen the target substrate.

It should be understood that, within the scope of the present disclosure, each of the above technical features of the present disclosure and each of the technical features specifically described hereinafter (e.g., embodiments) may be combined with each other, thereby constituting a further or preferable technical solution. For lack of space, it will not be repeated herein.

### BRIEF DESCRIPTION OF THE DRAWINGS

The foregoing and/or additional aspects and advantages of the present disclosure will become apparent and readily appreciated from the following descriptions made with reference to the drawings, in which:
Figure 1 is a schematic diagram showing a structure of a prokaryotic expression plasmid pET28a-Gluc-wt, which encodes wild type Gaussia luciferase with a signal peptide;
Figure 2 is a schematic diagram showing a structure of a prokaryotic expression plasmid pCold-no-sp-Gluc, which encodes wild type Gaussia luciferase without a signal peptide;
Figure 3 is a structural diagram of substrates of coelenterazine (A), fluoro-coelenterazine (B), coelenterazine derivative ZS2 (C), and coelenterazine derivative ZS26 (D);
Figure 4 shows an activity assay result of Gaussia luciferase at bacteria solution level;
Figure 5 shows results of expression and purification of Gaussia luciferase in different competent cells;
Figure 6 is a bar graph showing activities of Gaussia luciferase mutants on coelenterazine as a substrate determined at protein level, where "no sp Gluc" represents the wild type Gaussia luciferase without signal peptide, and having a sequence shown in SEQ ID NO: 3; and "no sp Gluc average" represents an average of groups of the wild type Gaussia luciferase without signal peptide and having the sequence shown in SEQ ID NO: 3, according to an embodiment of the present disclosure;
Figure 7 is a bar graph showing activities of Gaussia luciferase mutants on fluoro-coelenterazine as a substrate determined at protein level, where "no sp Gluc" represents the wild type Gaussia luciferase without signal peptide, and having a sequence shown in SEQ ID NO: 3, and "no sp Gluc average" represents an average of groups the wild type Gaussia luciferase without signal peptide and having the sequence shown in SEQ ID NO: 3, according to an embodiment of the present disclosure;
Figure 8 is a bar graph showing activities of advantageous Gaussia luciferase mutants on coelenterazine derivative ZS2 as a substrate at protein level, where "no sp Gluc" represents the wild type Gaussia luciferase without signal peptide, having a sequence shown in SEQ ID NO: 3, and "no sp Gluc average" represents an average of groups of the wild type Gaussia luciferase without signal peptide having the sequence shown in SEQ ID NO: 3, according to an embodiment of the present disclosure;
Figure 9 shows an absolute activity value (histogram) and a ratio of activity values (dot graph) of each Gaussia luciferase mutant on catalyzing fluoro-coelenterazine relative to its derivative ZS26 as determined at protein level, where the left ordinate presents luminescence (max), bar represents the absolute value of luminescence, and the right ordinate presents relative luminescence units (vs CTZ), dot represents the ratio of activity value, according to an embodiment of the present disclosure;
Figure 10 shows an absolute activity value (histogram) and a ratio of specific activity values (dot graph) of each Gaussia luciferase mutant on catalyzing a fluoro-coelenterazine derivative ZS2 relative to a coelenterazine derivative ZS26 as determined at protein level, where the left presents ordinate luminescence (max), bar represents the absolute value of luminescence, and the right ordinate presents relative luminescence units (vs CTZ), dot represents the ratio of activity values, according to an embodiment of the present disclosure;
Figure 11 is a schematic diagram showing a structure of a eukaryotic expression plasmid pEE12.4-Gluc, which encodes wild type Gaussia luciferase with a signal peptide, where "*signal peptide* " represents the signal peptide;
Figure 12 shows results of expression and purification of Gaussia luciferase in eukaryotic Expi CHO;
Figure 13 shows Gaussia luciferase coupled with SA protein before and after purification, where "-" represents before purification and "+" represents after purification; and
Figure 14 shows activities of the Gaussia luciferase coupled with SA protein to bind to different substrates, according to an embodiment of the present disclosure.

### DETAILED DESCRIPTION

Embodiments of the present disclosure will be described in detail below, and examples of embodiments are illustrated in the the drawings. Embodiments described herein with reference to the drawings are explanatory, serve to explain the present disclosure, and are not construed to limit embodiments of the present disclosure.

In addition, terms such as "first" and "second" are used herein for purposes of description and are not intended to indicate or imply relative importance or significance or impliedly indicate quantity of the technical feature referred to. Thus, the feature defined with "first" and "second" may include one or more this feature. In the description of the present disclosure, "a plurality of" means two or more than two this features, such as two or three, unless specified otherwise.

"Gaussia luciferase", as an enzyme for bioluminescence, is a protein with a molecular weight of 20kDa and derived from the *Gaussia princeps* of marine copepods. The Gaussia luciferase receptor is very small and independent of assistance of ATP, and could catalyze oxidation and luminescence of coelenterazine in the presence of oxygen molecules.

In the field of basic scientific research, a luciferase gene has been widely used as a reporter gene in the study of exogenous gene expression levels and transcriptional regulation under different promoters. In the field of biological monitoring, luciferase may be used to detect the presence or absence of chemical pollutants. In addition, it also has a broad application prospect in the field of immunoassay, biochemical diagnosis and so on. The present disclosure provides important mutation sites on wild type Gaussia luciferase, and these mutation sites have an important effect on enhancing protein performances.

### Mutant

In one aspect of the present disclosure, there is provided in embodiments a mutant. According to the embodiment of the present disclosure, the mutant includes at least one mutation, compared with an amino acid sequence as shown in SEQ ID NO: 3, occurred at a position(s) selected from positions 24, 26, 27, 29, 30, 31, 32, 33, 36, 37, 40, 66, 79, 84, 88, 102, 103, 104, 110, 123, 124, 138, 152, 163, 167, 170, 174, 175, 178, 182, and 183 thereof. According to the embodiment of the present disclosure, the mutants obtained by modifying the above discussed mutation sites have a strong catalytic activity on substrates such as coelenterazine, fluoro-coelenterazine, and a coelenterazine derivative ZS2, and has a wider substrate spectrum or higher substrate selectivity specificity and significantly enhanced luminescence brightness therein, compared with the existing Gaussia luciferase. Therefore, the mutant may be applied in the fields involving with luminescent detection, such as basic scientific research, biological detection, immune detection, biochemical detection, or diagnosis, and has broad application prospects. For example, the mutant may be used as a reporter gene to quantitatively detect DNA, RNA, transcription factors, proteins or cells; as well as may be used as a luminescent signal protein in a fusion protein to quantitatively detect target small molecules, proteins, etc.

Herein, the term "reporter gene" which is a molecular biology concept, refers to a class of genes that are expressed in cells, tissues/organs, or individuals under specific circumstances and enable them to produce easily detectable traits that would not otherwise be produced by the experimental material originally, i.e., a gene encoding a protein or enzyme that could be detected. A reporter gene should has the following features for genetic selection and screening: (1) the reporter gene has been cloned and fully sequenced; (2) the corresponding expression product thereof is not present in the recipient cell originally, i.e., there is no background or no endogenous expression product similar to such an expression product of the reporter gene in the transfected cell; and (3) the expression product of the reporter gene could be quantitatively determined, and the reporter gene may be used in a number of manners, including, but not limited to: fusing the reporter gene and a regulatory sequence or other target genes to form a chimeric gene, with nucleic acids expressing under the control of the regulatory sequence, so as to utilize the expression products of the reporter gene to detect the expression regulation of target genes, thereby researching the nucleic acids.

Herein, the term "nucleic acid expression" means that a DNA may be expressed as an RNA; or a DNA may be expressed as an RNA, and the RNA is further expressed as a protein; or an RNA may be expressed as a protein. In other words, the product of nucleic acid expression herein may be either an RNA or protein.

Herein, the term "chemiluminescence" is also called "cold light", which means that light radiation produced by a chemical reaction in the absence of excitation by light, heat or an electric field. There is also chemiluminescence presented in living systems, as termed as "bioluminescence", such as light emitted by firefly, certain bacteria or fungi, protozoa, worms and crustaceans. In embodiments of the present disclosure, the mutants are capable of self-luminescence, i.e. chemiluminescence.

According to specific embodiments of the present disclosure, the above mutants may further include at least one of the following additional technical features.

KPTENNEDFNIVAVASNFATTDLDADRGKLPGKKLPLEVLKEMEANARKAGCTRGC
LICLSHIKCTPKMKKFIPGRCHTYEGDKESAQGGIGEAIVDIPEIPGFKDLEPMEQFIAQVD
LCVDCTTGCLKGLANVQCSDLLKKWLPQRCATFASKIQGQVDKIKGAGGD (SEQ ID NO:
3)

According to specific embodiments of the present disclosure, the mutant includes at least one of the following mutations compared with the amino acid sequence as shown in SEQ ID NO: 3: 1) E at position 24 is mutated into K; 2) F at position 26 is mutated into R or L; 3) N at position 27 is mutated into D; 4) V at position 29 is mutated into F or L; 5) A at position 30 is mutated into G or D; 6) V at position 31 is mutated into I; 7) A at position 32 is mutated into V; 8) S at position 33 is mutated into E, R or K; 9) A at position 36 is mutated into V or I; 10) T at position 37 is mutated into N or E; 11) L at position 40 is mutated into I or T; 12) K at position 66 is mutated into P, S, I, R or N; 13) H at position 79 is mutated into K; 14) P at position 84 is mutated into A, L, K or V; 15) K at position 88 is mutated into R; 16) E at position 102 is mutated into D, A, S, K or N; 17) S at position 103 is mutated into T; 18) A at position 104 is mutated into G; 19) E at position 110 is mutated into P, G or A; 20) D at position 123 is mutated into N; 21) L at position 124 is mutated into M, G or I; 22) V at position 138 is mutated into E or D; 23) Q at position 152 is mutated into R or H; 24) Q at position 163 is mutated into D; 25) S at position 170 is mutated into N or T; 26) G at position 174 is mutated into K; 27) Q at position 175 is mutated into E; 28) K at position 178 is mutated into T; 29) A at position 182 is mutated into M; and 30) G at position 183 is mutated into N or A.

According to specific embodiments of the present disclosure, the mutant includes the following mutations compared with the amino acid sequence as shown in SEQ ID NO: 3, occurred at positions: 1) 79, 84, 102, 103, 104, 124 and 138; or 2) 66, 79, 84, 103, 104 and 138; or 3) 66, 79, 84, 102, 103, 104, 124 and 138; or 4) 79, 84, 102, 103, 104, 124 and 138; or 5) 66, 79, 102, 103, 104 and 138; or 6) 66, 79, 84, 102, 104, 124 and 138; or 7) 79, 84, 102, 104, 110 and 124; or 8) 84, 102, 104, 110 and 124; or 9) 66, 79, 84, 102, 103, 104, 110, 124 and 138; or 10) 66, 84, 102, 103, 104, 110, 124 and 138; or 11) 66, 84, 88, 102, 103, 104 and 124; or 12) 84, 102, 103, 104 and 110; or 13) 66, 79, 84, 102, 103, 104 and 138; or 14) 66, 84, 102, 103, 104, 110, 124 and 138; or 15) 66, 79, 102, 103, 104 and 138; or 16) 26, 29, 32, 33, 36, 40, 66, 79, 84, 102, 103, 104, 110, 124 and 138; or 17) 24, 79, 84, 102, 103, 104, 110, 124, 152, 163, 170, 175, 178, 182 and 183; or 18) 79, 84, 102, 103, 104, 110, 124, 152, 163, 167, 170, 174, 182 and 183; or 19) 79, 84, 102, 103, 104, 110, 124, 152, 163, 167, 170, 174, 182 and 183; or 20) 27, 29, 30, 32, 33, 36, 37, 40, 66, 79, 84, 102, 103, 104, 109, 110, 124 and 138; or 21) 26, 29, 30, 31, 33, 36, 37, 66, 79, 84, 102, 103, 104, 110, 124 and 138; or 22) 79, 102, 103, 104, 110, 124 and 138. According to the specific embodiment of the present disclosure, the mutant has a strong catalytic activity on substrates, and has a wider substrate spectrum or higher substrate selectivity specificity and significantly enhanced luminescence brightness therein, compared with the existing Gaussia luciferase, thereby significantly improving the detection accuracy in practical applications.

According to specific embodiments of the present disclosure, the mutant includes the following mutations compared with the amino acid sequence as shown in SEQ ID NO: 3 : 1) H at position 79 is mutated into K, P at position 84 is mutated into A, E at position 102 is mutated into D, S at position 103 is mutated into T, A at position 104 is mutated into G, L at position 124 is mutated into M, and V at position 138 is mutated into E; or 2) K at position 66 is mutated into R, H at position 79 is mutated into K, P at position 84 is mutated into L, S at position 103 is mutated into T, A at position 104 is mutated into G, and V at position 138 is mutated into E; or 3) K at position 66 is mutated into R, H at position 79 is mutated into K, P at position 84 is mutated into A, E at position 102 is mutated into A, S at position 103 is mutated into T, A at position 104 is mutated into G, L at position 124 is mutated into M, and V at position 138 is mutated into D; or 4) H at position 79 is mutated into K, P at position 84 is mutated into L, E at position 102 is mutated into S, S at position 103 is mutated into T, A at position 104 is mutated into G, L at position 124 is mutated into M, and V at position 138 is mutated into E; or 5) K at position 66 is mutated into N, H at position 79 is mutated into K, E at position 102 is mutated into A, S at position 103 is mutated into T, A at position 104 is mutated into G, and V at position 138 is mutated into E; or 6) K at position 66 is mutated into R, H at position 79 is mutated into K, P at position 84 is mutated into A, E at position 102 is mutated into S, A at position 104 is mutated into G, L at position 124 is mutated into M, and V at position 138 is mutated into E; or 7) H at position 79 is mutated into K, P at position 84 is mutated into A, E at position 102 is mutated into D, A at position 104 is mutated into G, E at position 110 is mutated into A, and L at position 124 is mutated into M; or 8) P at position 84 is mutated into A, E at position 102 is mutated into K, A at position 104 is mutated into G, E at position 110 is mutated into A, and L at position 124 is mutated into M; or 9) K at position 66 is mutated into N, H at position 79 is mutated into K, P at position 84 is mutated into L, E at position 102 is mutated into S, S at position 103 is mutated into T, A at position 104 is mutated into G, E at position 110 is mutated into P, L at position 124 is mutated into M, and V at position 138 is mutated into D; or 10) K at position 66 is mutated into R, P at position 84 is mutated into A, E at position 102 is mutated into A, S at position 103 is mutated into T, A at position 104 is mutated into G, E at position 110 is mutated into P, L at position 124 is mutated into M, and V at position 138 is mutated into E; or 11) K at position 66 is mutated into R, P at position 84 is mutated into L, K at position 88 is mutated into R, E at position 102 is mutated into A, S at position 103 is mutated into T, A at position 104 is mutated into G, and L at position 124 is mutated into M; 12) P at position 84 is mutated into K, E at position 102 is mutated into A, S at position 103 is mutated into T, A at position 104 is mutated into G, and V at position 138 is mutated into D; or 13) K at position 66 is mutated into N, H at position 79 is mutated into K, P at position 84 is mutated into A, E at position 102 is mutated into D, S at position 103 is mutated into T, A at position 104 is mutated into G, and V at position 138 is mutated into D; or 14) K at position 66 is mutated into N, P at position 84 is mutated into V, E at position 102 is mutated into N, S at position 103 is mutated into T, A at position 104 is mutated into G, E at position 110 is mutated into A, L at position 124 is mutated into M, and V at position 138 is mutated into D; or 15) K at position 66 is mutated into N, H at position 79 is mutated into K, E at position 102 is mutated into A, S at position 103 is mutated into T, A at position 104 is mutated into G, and V at position 138 is mutated into D; or 16) F at position 26 is mutated into R, V at position 29 is mutated into F, A at position 32 is mutated into V, S at position 33 is mutated into E, A at position 36 is mutated into V, L at position 40 is mutated into I, K at position 66 is mutated into P, H at position 79 is mutated into K, P at position 84 is mutated into L, E at position 102 is mutated into S, S at position 103 is mutated into T, A at position 104 is mutated into G, E at position 110 is mutated into P L at position 124 is mutated into M and V at position 138 is mutated into E; or 17) E at position 24 is mutated into K, H at position 79 is mutated into K, P at position 84 is mutated into L, E at position 102 is mutated into S, S at position 103 is mutated into T, A at position 104 is mutated into G, E at position 110 is mutated into P, L at position 124 is mutated into G, Q at position 152 is mutated into R, Q at position 163 is mutated into D, S at position 170 is mutated into N, Q at position 175 is mutated into E, K at position 178 is mutated into T, A at position 182 is mutated into M and G at position 183 is mutated into N; or 18) H at position 79 is mutated into K, P at position 84 is mutated into L, E at position 102 is mutated into S, S at position 103 is mutated into T, A at position 104 is mutated into G, E at position 110 is mutated into P, L at position 124 is mutated into I, Q at position 152 is mutated into H, Q at position 163 is mutated into D, T at position 167 is mutated into S, S at position 170 is mutated into N, G at position 174 is mutated into K, A at position 182 is mutated into M and G at position 183 is mutated into A; or 19) H at position 79 is mutated into K, P at position 84 is mutated into L, E at position 102 is mutated into S, S at position 103 is mutated into T, A at position 104 is mutated into G, E at position 110 is mutated into P, L at position 124 is mutated into M, Q at position 152 is mutated into H, Q at position 163 is mutated into D, T at position 167 is mutated into S, S at position 170 is mutated into T, G at position 174 is mutated into K, A at position 182 is mutated into M and G at position 183 is mutated into A; or 20) N at position 27 is mutated into D, V at position 29 is mutated into L, A at position 30 is mutated into G, A at position 32 is mutated into V, S at position 33 is mutated into R, A at position 36 is mutated into L, T at position 37 is mutated into N, L at position 40 is mutated into T, K at position 66 is mutated into S, H at position 79 is mutated into K, P at position 84 is mutated into L, E at position 102 is mutated into S, S at position 103 is mutated into T A at 104 is mutated into G, G at 109 is mutated into V, E at 110 is mutated into P, L at 124 is mutated into M, and V at 138 is mutated into E; or 21) F at position 26 is mutated into L, V at position 29 is mutated into L, A at position 30 is mutated into D, V at position 31 is mutated into I, S at position 33 is mutated into K, A at position 36 is mutated into I, T at position 37 is mutated into E, K at position 40 is mutated into I, K at position 66 is mutated into I, H at position 79 is mutated into K, P at position 84 is mutated into L, E at position 102 is mutated into S, S at position 103 is mutated into T, A at position 104 is mutated into G, G at position 109 is mutated into V, E at position 110 is mutated into P, L at position 124 is mutated into M, and V at position 138 is mutated into E; or 22) H at position 79 is mutated into K, E at position 102 is mutated into S, S at position 103 is mutated into T, A at position 104 is mutated into G, E at position 110 is mutated into P, L at position 124 is mutated into M, and V at position 138 is mutated into E. According to some specific embodiments of the present disclosure, when the amino acid sequence shown in SEQ ID NO: 3 includes the mutation as described above, the corresponding protein has a strong catalytic activity on substrates such as coelenterazine, fluoro-coelenterazine and coelenterazine derivatives, and has a wider substrate spectrum or higher substrate selectivity specificity and significantly enhanced luminescence brightness therein, compared with the existing Gaussia luciferase, thereby significantly improving the detection accuracy in practical applications. Therefore, the mutant protein may be applied in the fields involving with luminescent detection, such as basic scientific research, biological detection, immune detection, biochemical detection, or diagnosis, and has broad application prospects.

According to some specific embodiments of the present disclosure, the mutant is a non-secretory protein or a secretory protein.

### Preparation of mutants

In another aspect, the present disclosure provides in embodiments a nucleic acid molecule that encodes the mutant described above. The nucleic acid molecule according to the specific embodiments of the present disclosure could be used as a reporter gene. In addition, the mutant encoded by the nucleic acid molecule has a strong catalytic activity on substrates such as coelenterazine, fluoro-coelenterazine and coelenterazine derivatives, and has a wider substrate spectrum or higher substrate selectivity specificity and significantly enhanced luminescence brightness therein compared with the existing Gaussia luciferase, thereby significantly improving the detection accuracy in practical applications. Therefore, the mutant protein may be applied in the fields involving with luminescent detection, such as basic scientific research, biological detection, immune detection, biochemical detection, or diagnosis, and has broad application prospects.

According to some specific embodiments of the present disclosure, the above-described nucleic acid molecule may further include at least one of the following additional technical features.

According to specific embodiments of the present disclosure, the nucleic acid molecule has a nucleotide sequence as shown in any one of SEQ ID NOs: 22-43.

A mutant D6 is encoded by a gene with the following nucleotide sequence:

ATGAAACCAACTGAAAACAATGAAGATTTCAACATTGTAGCTGTAGCTAGCAACT
TTGCTACAACGGATCTCGATGCTGACCGTGGTAAATTGCCCGGAAAAAATTACCACT
TGAGGTACTCAAAGAAATGGAAGCCAATGCTAGGAAAGCTGGCTGCACTAGGGGATG
TCTGATATGCCTGTCAAAAATCAAGTGTACAGCCAAAATGAAGAAGTTTATCCCAGGA
AGATGCCACACCTATGAAGGAGACAAAGACACTGGACAGGGAGGAATAGGAGAGGC
TATTGTTGACATTCCTGAAATTCCTGGGTTTAAGGACATGGAACCCATGGAACAATTCA
TTGCACAAGTTGACCTATGTGAAGACTGCACAACTGGATGCCTCAAAGGTCTTGCCA
ATGTGCAATGTTCTGATTTACTCAAGAAATGGCTGCCACAAAGATGTGCAACTTTTGC
TAGCAAAATTCAAGGCCAAGTGGACAAAATAAAGGGTGCCGGTGGTGAT (SEQ ID NO:
22).

A mutant E7 is encoded by a gene with the following nucleotide sequence:

ATGAAACCAACTGAAAACAATGAAGATTTCAACATTGTAGCTGTAGCTAGCAACT
TTGCTACAACGGATCTCGATGCTGACCGTGGTAAATTGCCCGGAAAAAATTACCACT
TGAGGTACTCAAAGAAATGGAAGCCAATGCTAGGAGAGCTGGCTGCACTAGGGGATG
TCTGATATGCCTGTCAAAAATCAAGTGTACACTCAAAATGAAGAAGTTTATCCCAGGA
AGATGCCACACCTATGAAGGAGACAAAGAAACTGGACAGGGAGGAATAGGAGAGGC
TATTGTTGACATTCCTGAAATTCCTGGGTTTAAGGACTTGGAACCCATGGAACAATTCA
TTGCACAAGTTGACCTATGTGAAGACTGCACAACTGGATGCCTCAAAGGTCTTGCCA
ATGTGCAATGTTCTGATTTACTCAAGAAATGGCTGCCACAAAGATGTGCAACTTTTGC
TAGCAAAATTCAAGGCCAAGTGGACAAAATAAAGGGTGCCGGTGGTGAT (SEQ ID NO:
23).

A mutant C10 is encoded by a gene with the following nucleotide sequence:

ATGAAACCAACTGAAAACAATGAAGATTTCAACATTGTAGCTGTAGCTAGCAACT
TTGCTACAACGGATCTCGATGCTGACCGTGGTAAATTGCCCGGAAAAAATTACCACT
TGAGGTACTCAAAGAAATGGAAGCCAATGCTAGGAGAGCTGGCTGCACTAGGGGATG
TCTGATATGCCTGTCAAAAATCAAGTGTACAGCCAAAATGAAGAAGTTTATCCCAGGA
AGATGCCACACCTATGAAGGAGACAAAGCAACTGGACAGGGAGGAATAGGAGAGGC
TATTGTTGACATTCCTGAAATTCCTGGGTTTAAGGACATGGAACCCATGGAACAATTCA
TTGCACAAGTTGACCTATGTGATGACTGCACAACTGGATGCCTCAAAGGTCTTGCCAA
TGTGCAATGTTCTGATTTACTCAAGAAATGGCTGCCACAAAGATGTGCAACTTTTGCT
AGCAAAATTCAAGGCCAAGTGGACAAAATAAAGGGTGCCGGTGGTGAT (SEQ ID NO:
24).

A mutant B6 is encoded by a gene with the following nucleotide sequence:

ATGAAACCAACTGAAAACAATGAAGATTTCAACATTGTAGCTGTAGCTAGCAACT
TTGCTACAACGGATCTCGATGCTGACCGTGGTAAATTGCCCGGAAAAAATTACCACT
TGAGGTACTCAAAGAAATGGAAGCCAATGCTAGGAAAGCTGGCTGCACTAGGGGATG
TCTGATATGCCTGTCAAAAATCAAGTGTACACTCAAAATGAAGAAGTTTATCCCAGGA
AGATGCCACACCTATGAAGGAGACAAATCAACTGGACAGGGAGGAATAGGACCGGCT
ATTGTTGACATTCCTGAAATTCCTGGGTTTAAGGATATGGAACCCATGGAACAATTCAT
TGCACAAGTTGACCTATGTGAAGACTGCACAACTGGATGCCTCAAAGGTCTTGCCAAT
GTGCAATGTTCTGATTTACTCAAGAAATGGCTGCCACAAAGATGTGCAACTTTTGCTA
GCAAAATTCAAGGCCAAGTGGACAAAATAAAGGGTGCCGGTGGTGAT (SEQ ID NO:
25).

A mutant E12 is encoded by a gene with the following nucleotide sequence:

ATGAAACCAACTGAAAACAATGAAGATTTCAACATTGTAGCTGTAGCTAGCAACT
TTGCTACAACGGATCTCGATGCTGACCGTGGTAAATTGCCCGGAAAAAATTACCACT
TGAGGTACTCAAAGAAATGGAAGCCAATGCTAGGAACGCTGGCTGCACTAGGGGATG
TCTGATATGCCTGTCAAAAATCAAGTGTACACTCAAAATGAAGAAGTTTATCCCAGGA
AGATGCCACACCTATGAAGGAGACAAAGCAACTGGACAGGGAGGAATAGGAGAGGC
TATTGTTGACATTCCTGAAATTCCTGGGTTTAAGGACTTGGAACCCATGGAACAATTCA
TTGCACAAGTTGACCTATGTGAAGACTGCACAACTGGATGCCTCAAAGGTCTTGCCA
ATGTGCAATGTTCTGATTTACTCAAGAAATGGCTGCCACAAAGATGTGCAACTTTTGC
TAGCAAAATTCAAGGCCAAGTGGACAAAATAAAGGGTGCCGGTGGTGAT (SEQ ID NO:
26).

A mutant NO. 1 is encoded by a gene with the following nucleotide sequence:

ATGAAACCAACTGAAAACAATGAAGATTTCAACATTGTAGCTGTAGCTAGCAACT
TTGCTACAACGGATCTCGATGCTGACCGTGGTAAATTGCCCGGAAAAAATTACCACT
TGAGGTACTCAAAGAAATGGAAGCCAATGCTAGGAGAGCTGGCTGCACTAGGGGATG
TCTGATATGCCTGTCAAAAATCAAGTGTACAGCCAAAATGAAGAAGTTTATCCCAGGA
AGATGCCACACCTATGAAGGAGACAAATCAAGTGGACAGGGAGGAATAGGAGAGGC
TATTGTTGACATTCCTGAAATTCCTGGGTTTAAGGACATGGAACCCATGGAACAATTCA
TTGCACAAGTTGACCTATGTGAAGACTGCACAACTGGATGCCTCAAAGGTCTTGCCA
ATGTGCAATGTTCTGATTTACTCAAGAAATGGCTGCCACAAAGATGTGCAACTTTTGC
TAGCAAAATTCAAGGCCAAGTGGACAAAATAAAGGGTGCCGGTGGTGAT (SEQ ID NO:
27).

A mutant NO. 11 is encoded by a gene with the following nucleotide sequence:

ATGAAACCAACTGAAAACAATGAAGATTTCAACATTGTAGCTGTAGCTAGCAACT
TTGCTACAACGGATCTCGATGCTGACCGTGGTAAATTGCCCGGAAAAAATTACCACT
TGAGGTACTCAAAGAAATGGAAGCCAATGCTAGGAAAGCTGGCTGCACTAGGGGATG
TCTGATATGCCTGTCAAAAATCAAGTGTACAGCCAAAATGAAGAAGTTTATCCCAGGA
AGATGCCACACCTATGAAGGAGACAAAGACAGTGGACAGGGAGGAATAGGAGCGGC
TATTGTTGACATTCCTGAAATTCCTGGGTTTAAGGATATGGAACCCATGGAACAATTCA
TTGCACAAGTTGACCTATGTGTAGACTGCACAACTGGATGCCTCAAAGGTCTTGCCAA
TGTGCAATGTTCTGATTTACTCAAGAAATGGCTGCCACAAAGATGTGCAACTTTTGCT
AGCAAAATTCAAGGCCAAGTGGACAAAATAAAGGGTGCCGGTGGTGAT (SEQ ID NO:
28).

A mutant E11 is encoded by a gene with the following nucleotide sequence:

ATGAAACCAACTGAAAACAATGAAGATTTCAACATTGTAGCTGTAGCTAGCAACT
TTGCTACAACGGATCTCGATGCTGACCGTGGTAAATTGCCCGGAAAAAATTACCACT
TGAGGTACTCAAAGAAATGGAAGCCAATGCTAGGAAAGCTGGCTGCACTAGGGGATG
TCTGATATGCCTGTCACACATCAAGTGTACAGCCAAAATGAAGAAGTTTATCCCAGGA
AGATGCCACACCTATGAAGGAGACAAAAAAAGTGGACAGGGAGGAATAGGAGCGGC
TATTGTTGACATTCCTGAAATTCCTGGGTTTAAGGATATGGAACCCATGGAACAATTCA
TTGCACAAGTTGACCTATGTGTAGACTGCACAACTGGATGCCTCAAAGGTCTTGCCAA
TGTGCAATGTTCTGATTTACTCAAGAAATGGCTGCCACAAAGATGTGCAACTTTTGCT
AGCAAAATTCAAGGCCAAGTGGACAAAATAAAGGGTGCCGGTGGTGAT (SEQ ID NO:
29).

A mutant NO. 23 is encoded by a gene with the following nucleotide sequence:

ATGAAACCAACTGAAAACAATGAAGATTTCAACATTGTAGCTGTAGCTAGCAACT
TTGCTACAACGGATCTCGATGCTGACCGTGGTAAATTGCCCGGAAAAAATTACCACT
TGAGGTACTCAAAGAAATGGAAGCCAATGCTAGGAACGCTGGCTGCACTAGGGGATG
TCTGATATGCCTGTCAAAAATCAAGTGTACACTCAAAATGAAGAAGTTTATCCCAGGA
AGATGCCACACCTATGAAGGAGACAAATCAACTGGACAGGGAGGAATAGGACCGGCT
ATTGTTGACATTCCTGAAATCCCTGGGTTTAAGGATATGGAACCCATGGAACAATTCAT
TGCACAAGTTGACCTATGTGATGACTGCACAACTGGATGCCTCAAAGGTCTTGCCAAT
GTGCAATGTTCTGATTTACTCAAGAAATGGCTGCCACAAAGATGTGCAACTTTTGCTA
GCAAAATTCAAGGCCAAGTGGACAAAATAAAGGGTGCCGGTGGTGAT (SEQ ID NO:
30).

A mutant B7 is encoded by a gene with the following nucleotide sequence:

ATGAAACCAACTGAAAACAATGAAGATTTCAACATTGTAGCTGTAGCTAGCAACT

TTGCTACAACGGATCTCGATGCTGACCGTGGTAAATTGCCCGGAAAAAAATTACCACT
TGAGGTACTCAAAGAAATGGAAGCCAATGCTAGGAGAGCTGGCTGCACTAGGGGATG
TCTGATATGCCTGTCACACATCAAGTGTACAGCCAAAATGAAGAAGTTTATCCCAGGA
AGATGCCACACCTATGAAGGAGACAAAGCAACTGGACAGGGAGGAATAGGACCGGC
TATTGTTGACATTCCTGAAATTCCTGGGTTTAAGGATATGGAACCCATGGAACAATTCA
TTGCACAAGTTGACCTATGTGAAGACTGCACAACTGGATGCCTCAAAGGTCTTGCCA
ATGTGCAATGTTCTGATTTACTCAAGAAATGGCTGCCACAAAGATGTGCAACTTTTGC
TAGCAAAATTCAAGGCCAAGTGGACAAAATAAAGGGTGCCGGTGGTGAT (SEQ ID NO:
31).

A mutant A9 is encoded by a gene with the following nucleotide sequence:

ATGAAACCAACTGAAAACAATGAAGATTTCAACATTGTAGCTGTAGCTAGCAACT
TTGCTACAACGGATCTCGATGCTGACCGTGGTAAATTGCCCGGAAAAAATTACCACT
TGAGGTACTCAAAGAAATGGAAGCCAATGCTAGGAGAGCTGGCTGCACTAGGGGATG
TCTGATATGCCTGTCACACATCAAGTGTACACTCAAAATGAAGAGGTTTATCCCAGGA
AGATGCCACACCTATGAAGGAGACAAAGCAACTGGACAGGGAGGAATAGGAGAGGC
TATTGTTGACATTCCTGAAATTCCTGGGTTTAAGGATATGGAACCCATGGAACAATTCA
TTGCACAAGTTGACCTATGTGTAGACTGCACAACTGGATGCCTCAAAGGTCTTGCCAA
TGTGCAATGTTCTGATTTACTCAAGAAATGGCTGCCACAAAGATGTGCAACTTTTGCT
AGCAAAATTCAAGGCCAAGTGGACAAAATAAAGGGTGCCGGTGGTGAT (SEQ ID NO:
32).

A mutant G2 is encoded by a gene with the following nucleotide sequence:

ATGAAACCAACTGAAAACAATGAAGATTTCAACATTGTAGCTGTAGCTAGCAACT
TTGCTACAACGGATCTCGATGCTGACCGTGGTAAATTGCCCGGAAAAAATTACCACT
TGAGGTACTCAAAGAAATGGAAGCCAATGCTAGGAACGCTGGCTGCACTAGGGGATG
TCTGATATGCCTGTCACACATCAAGTGTACAAAGAAAATGAAGAAGTTTATCCCAGGA
AGATGCCACACCTATGAAGGAGACAAAGAAACTGGACAGGGAGGAATAGGAGGGGC
TATTGTTGACATTCCTGAAATTCCTGGGTTTAAGAACATGGAACCCATGGAACAATTCA
TTGCACAAGTTGACCTATGTGAAGACTGCACAACTGGATGCCTCAAAGGTCTTGCCA
ATGTGCAATGTTCTGATTTACTCAAGAAATGGCTGCCACAAAGATGTGCAACTTTTGC
TAGCAAAATTCAAGGCCAAGTGGACAAAATAAAGGGTGCCGGTGGTGAT (SEQ ID NO:
33).

A mutant E8 is encoded by a gene with the following nucleotide sequence:

ATGAAACCAACTGAAAACAATGAAGATTTCAACATTGTAGCTGTAGCTAGCAACT
TTGCTACAACGGATCTCGATGCTGACCGTGGTAAATTGCCCGGAAAAAATTACCACT

TGAGGTACTCAAAGAAATGGAAGCCAATGCTAGGAAAGCTGGCTGCACTAGGGGATG
TCTGATATGCCTGTCACACATCAAGTGTACAAAGAAAATGAAGAAGTTTATCCCAGGA
AGATGCCACACCTATGAAGGAGACAAAGCAACTGGACAGGGAGGAATAGGACCGGC
TATTGTTGACATTCCTGAAATTCCTGGGTTTAAGGATTTGGAACCCATGGAACAATTCA
TTGCACAAGTTGACCTATGTGTAGACTGCACAACTGGATGCCTCAAAGGTCTTGCCAA
TGTGCAATGTTCTGATTTACTCAAGAAATGGCTGCCACAAAGATGTGCAACTTTTGCT
AGCAAAATTCAAGGCCAAGTGGACAAAATAAAGGGTGCCGGTGGTGAT (SEQ ID NO:
34).

A mutant NO. 15 is encoded by a gene with the following nucleotide sequence:

ATGAAACCAACTGAAAACAATGAAGATTTCAACATTGTAGCTGTAGCTAGCAACT
TTGCTACAACGGATCTCGATGCTGACCGTGGTAAATTGCCCGGAAAAAATTACCACT
TGAGGTACTCAAAGAAATGGAAGCCAATGCTAGGAACGCTGGCTGCACTAGGGGATG
TCTGATATGCCTGTCAAAAATCAAGTGTACAGCCAAAATGAAGAAGTTTATCCCAGGA
AGATGCCACACCTATGAAGGAGACAAAGACACTGGACAGGGAGGAATAGGAGAGGC
TATTGTTGACATTCCTGAAATTCCTGGGTTTAAGGACTTGGAACCCATGGAACAATTCA
TTGCACAAGTTGACCTATGTGATGACTGCACAACTGGATGCCTCAAAGGTCTTGCCAA
TGTGCAATGTTCTGATTTACTCAAGAAATGGCTGCCACAAAGATGTGCAACTTTTGCT
AGCAAAATTCAAGGCCAAGTGGACAAAATAAAGGGTGCCGGTGGTGAT (SEQ ID NO:
35).

A mutant A7 is encoded by a gene with the following nucleotide sequence:

AAACCAACTGAAAACAATGAAGATTTCAACATTGTAGCTGTAGCTAGCAACTTTG
CTACAACGGATCTCGATGCTGACCGTGGTAAATTGCCCGGAAAAAATTACCACTTGA
GGTACTCAAAGAAATGGAAGCCAATGCTAGGAACGCTGGCTGCACTAGGGGATGTCT
GATATGCCTGTCACACATCAAGTGTACAGTCAAAATGAAGAAGTTTATCCCAGGAAGA
TGCCACACCTATGAAGGAGACAAAAACACTGGACAGGGAGGAATAGGAGCGGCTATT
GTTGACATTCCTGAAATTCCTGGGTTTAAGGACATGGAACCCATGGAACAATTCATTG
CACAAGTTGACCTATGTGATGACTGCACAACTGGATGCCTCAAAGGTCTTGCCAATGT
GCAATGTTCTGATTTACTCAAGAAATGGCTGCCACAAAGATGTGCAACTTTTGCTAGC
AAAATTCAAGGCCAAGTGGACAAAATAAAGGGTGCCGGTGGTGAT (SEQ ID NO: 36).

A mutant E1-A3 is encoded by a gene with the following nucleotide sequence:

ATGAAACCAACTGAAAACAATGAAGATCGCAACATTTTCGCTGTAGTTGAGAACT
TTGTTACGACGGATATTGATGCTGACCGTGGTAAATTGCCCGGAAAAAAATTACCACT
TGAGGTACTCAAAGAAATGGAAGCCAATGCTAGGCCTGCTGGCTGCACTAGGGGATG
TCTGATATGCCTGTCAAAAATCAAGTGTACACTCAAAATGAAGAAGTTTATCCCAGGA

AGATGCCACACCTATGAAGGAGACAAATCAACTGGACAGGGAGGAATAGGACCGGCT
ATTGTTGACATTCCTGAAATTCCTGGGTTTAAGGATATGGAACCCATGGAACAATTCAT
TGCACAAGTTGACCTATGTGAAGACTGCACAACTGGATGCCTCAAAGGTCTTGCCAAT
GTGCAATGTTCTGATTTACTCAAGAAATGGCTGCCACAAAGATGTGCAACTTTTGCTA
GCAAAATTCAAGGCCAAGTGGACAAAATAAAGGGTGCCGGTGGTGAT (SEQ ID NO:
37).

A mutant G2-F11 is encoded by a gene with the following nucleotide sequence:

ATGAAACCAACTGAAAACAATAAAGATTTCAACATTGTAGCTGTAGCTAGCAACT
TTGCTACAACGGATCTCGATGCTGACCGTGGTAAATTGCCCGGAAAAAATTACCACT
TGAGGTACTCAAAGAAATGGAAGCCAATGCTAGGAAAGCTGGCTGCACTAGGGGATG
TCTGATATGCCTGTCAAAAATCAAGTGTACACTCAAAATGAAGAAGTTTATCCCAGGA
AGATGCCACACCTATGAAGGAGACAAATCAACTGGACAGGGAGGAATAGGACCGGCT
ATTGTTGACATTCCTGAAATTCCTGGGTTTAAGGATGGGGAACCCATGGAACAATTCAT
TGCACAAGTTGACCTATGTGTAGACTGCACAACTGGATGCCTCAAAGGTCTTGCCAAT
GTGCGATGTTCTGATTTACTCAAGAAATGGCTGCCAGACAGATGTGCAACTTTTGCTA
ACAAAATTCAAGGCGAAGTGGACACAATAAAGGGTATGAACGGTGAT (SEQ ID NO: 38).

A mutant G2-E1 is encoded by a gene with the following nucleotide sequence:

ATGAAACCAACTGAAAACAATGAAGATTTCAACATTGTAGCTGTAGCTAGCAACT
TTGCTACAACGGATCTCGATGCTGACCGTGGTAAATTGCCCGGAAAAAATTACCACT
TGAGGTACTCAAAGAAATGGAAGCCAATGCTAGGAAAGCTGGCTGCACTAGGGGATG
TCTGATATGCCTGTCAAAAATCAAGTGTACACTCAAAATGAAGAAGTTTATCCCAGGA
AGATGCCACACCTATGAAGGAGACAAATCAACTGGACAGGGAGGAATAGGACCGGCT
ATTGTTGACATTCCTGAAATTCCTGGGTTTAAGGATATTGAACCCATGGAACAATTCAT
TGCACAAGTTGACCTATGTGTAGACTGCACAACTGGATGCCTCAAAGGTCTTGCCAAT
GTGCATTGTTCTGATTTACTCAAGAAATGGCTGCCAGACAGATGTGCAGGTTTTGCTA
ACAAAATTCAAAGCCAAGTGGACAACATAAAGGGTCTCGGTGGTGAT (SEQ ID NO: 39).

A mutant G2-F8 is encoded by a gene with the following nucleotide sequence:

ATGAAACCAACTGAAAACAATGAAGATTTCAACATTGTAGCTGTAGCTAGCAACT
TTGCTACAACGGATCTCGATGCTGACCGTGGTAAATTGCCCGGAAAAAAATTACCACT
TGAGGTACTCAAAGAAATGGAAGCCAATGCTAGGAAAGCTGGCTGCACTAGGGGATG
TCTGATATGCCTGTCAAAAATCAAGTGTACACTCAAAATGAAGAAGTTTATCCCAGGA
AGATGCCACACCTATGAAGGAGACAAATCAACTGGACAGGGAGGAATAGGACCGGCT
ATTGTTGACATTCCTGAAATTCCTGGGTTTAAGGATATGGAACCCATGGAACAATTCAT
TGCACAAGTTGACCTATGTGTAGACTGCACAACTGGATGCCTCAAAGGTCTTGCCAAT

GTGCATTGTTCTGATTTACTCAAGAAATGGCTGCCAGACAGATGTGCAAGTTTTGCTA
CCAAAATTCAAAAGCAAGTGGACAAAATAAAGGGTATGGCTGGTGAT (SEQ ID NO: 40).

A mutant E1-B4 is encoded by a gene with the following nucleotide sequence:

ATGAAACCAACTGAAAACAATGAAGATTTCGACATTTTGGGTGTAGTTAGGAACT
TTATTAACACGGATACCGATGCTGACCGTGGTAAATTGCCCGGAAAAAATTACCACT
TGAGGTACTCAAAGAAATGGAAGCCAATGCTAGGTCGGCTGGCTGCACTAGGGGATG
TCTGATATGCCTGTCAAAAATCAAGTGTACACTCAAAATGAAGAAGTTTATCCCAGGA
AGATGCCACACCTATGAAGGAGACAAATCAACTGGACAGGGAGGAATAGTACCGGCT
ATTGTTGACATTCCTGAAATTCCTGGGTTTAAGGATATGGAACCCATGGAACAATTCAT
TGCACAAGTTGACCTATGTGAAGACTGCACAACTGGATGCCTCAAAGGTCTTGCCAAT
GTGCAATGTTCTGATTTACTCAAGAAATGGCTGCCACAAAGATGTGCAACTTTTGCTA
GCAAAATTCAAGGCCAAGTGGACAAAATAAAGGGTGCCGGTGGTGAT (SEQ ID NO:
41).

A mutant E1-G12 is encoded by a gene with the following nucleotide sequence:

ATGAAACCAACTGAAAACAATGAAGATCTCAACATTTTAGATATAGCTAAGAACT
TTATTGAAACGGATCTTGATGCTGACCGTGGTAAATTGCCCGGAAAAAATTACCACT
TGAGGTACTCAAAGAAATGGAAGCCAATGCTAGGATTGCTGGCTGCACTAGGGGATG
TCTGATATGCCTGTCAAAAATCAAGTGTACACTCAAAATGAAGAAGTTTATCCCAGGA
AGATGCCACACCTATGAAGGAGACAAATCAACTGGACAGGGAGGAATAGGACCGGCT
ATTGTTGACATTCCTGAAATTCCTGGGTTTAAGGATATGGAACCCATGGAACAATTCAT
TGCACAAGTTGACCTATGTGAAGACTGCACAACTGGATGCCTCAAAGGTCTTGCCAAT
GTGCAATGTTCTGATTTACTCAAGAAATGGCTGCCACAAAGATGTGCAACTTTTGCTA
GCAAAATTCAAGGCCAAGTGGACAAAATAAAGGGTGCCGGTGGTGAT (SEQ ID NO:
42).

A mutant 4-C12 is encoded by a gene with the following nucleotide sequence:

ATGAAACCAACTGAAAACAATGAAGATTTCAACATTGTAGCTGTAGCTAGCAACT
TTGCTACAACGGATCTCGATGCTGACCGTGGTAAATTGCCCGGAAAAAATTACCACT
TGAGGTACTCAAAGAAATGGAAGCCAATGCTAGGAAAGCTGGCTGCACTAGGGGATG
TCTGATATGCCTGTCAAAGATCAAGTGTACACCCAAAATGAAGAAGTTTATCCCAGGA
AGATGCCACACCTATGAAGGAGACAAATCAACTGGACAGGGAGGAATAGGACCGGCT
ATTGTTGACATTCCTGAAATTCCTGGGTTTAAGGATATGGAACCCATGGAACAATTCAT
TGCACAAGTTGACCTATGTGAAGACTGCACAACTGGATGCCTCAAAGGTCTTGCCAAT
GTGCAATGTTCTGATTTACTCAAGAAATGGCTGCCACAAAGATGTGCAACTTTTGCTA
GCAAAATTCAAGGCCAAGTGGACAAAATAAAGGGTGCCGGTGGTGAT (SEQ ID NO:

43).

It should be noted that as understood by those skilled in the art, the nucleic acid mentioned in the description and claims of the present disclosure actually includes either one or both of the complementary double strands. Although only one strand is provided in most cases in the description and claims of the present disclosure for convenience, the other complementary strand is also actually considered to be disclosed. In addition, the nucleic acid sequence in the embodiments of the present disclosure includes its DNA form or RNA form, and one of which is disclosed means that the other is also disclosed.

In still another aspect, the present disclosure provides in embodiments an expression vector including the nucleic acid molecule described above. There is no specific limitation to types of the expression vector herein, as long as one is able to replicate and express the corresponding mutant in a host cell. The expression vector may include an optional control sequence, which is operably connected with the nucleic acid molecule. The control sequence refers to one or more control sequences that guides an expression of the nucleic acid molecule in the host. The expression vector provided in specific embodiments of the present disclosure may efficiently express a protein in suitable host cells, and the obtained protein has a strong catalytic activity on substrates such as coelenterazine, fluoro-coelenterazine and coelenterazine derivatives, and has a wider substrate spectrum or higher substrate selectivity specificity and significantly enhanced luminescence brightness therein, compared with the existing Gaussia luciferase. Therefore, the mutant protein may be applied in the fields involving with luminescent detection, such as basic scientific research, biological detection, immune detection, biochemical detection, or diagnosis, and has broad application prospects.

In yet another aspect, the present disclosure provides in embodiments a recombinant cell that carries the nucleic acid molecule, expression vector or mutant as described above. The recombinant cell is obtained by transfecting or transforming the expression vector. According to specific embodiments of the present disclosure, the recombinant cell may efficiently express the above mutant under appropriate conditions, and the mutant has a strong catalytic activity on substrates such as coelenterazine, fluoro-coelenterazine and coelenterazine derivatives, and has a wider substrate spectrum or higher substrate selectivity specificity and significantly enhanced luminescence brightness therein, compared with the existing Gaussia luciferase. Therefore, the mutant protein may be applied in the fields involving with luminescent detection, such as basic scientific research, biological detection, immune detection, biochemical detection, or diagnosis, and has broad application prospects.

It should be noted that the term "suitable conditions" in the specification of the present application refers to conditions suitable for expressing the mutant described herein. It is readily understood by those skilled in the art that the conditions suitable for expressing the mutant include, but are not limited to, a suitable transformation or transfection approach, suitable transformation or transfection conditions, a healthy state of host cell, a suitable density of host cell, a suitable cell culture environment, and a suitable cell culture time. The term "suitable conditions" are not particularly limited, and any one skilled in the art may optimize the most suitable conditions for the expression of the mutant according to the specific environment of the laboratory.

According to specific embodiments of the present disclosure, the recombinant cell may further includes at least one of the following additional technical features.

According to some specific embodiments of the present disclosure, the recombinant cell is *Escherichia coli, Saccharomyces cerevisiae,* an insect cell or a mammalian cell. According to specific embodiments of the present disclosure, the recombinant cell is not specifically limited and any cell with which the mutant contained in a nucleic acid or vector (e.g., a plasmid) is able to be expressed may be used, for example, a yeast cell, a bacterial cell, an insect cell, or a mammalian cell such as a human embryonic kidney cell.

According to specific embodiments of the present disclosure, the recombinant cell does not include animal germ cells, fertilized eggs or embryonic stem cells.

### Use

In one aspect, the present disclosure provides in embodiments use of the mutant as described above in the preparation of a kit for detecting a content of analyte, where a specific recognition protein of the analyte allows to form a complex with the mutant. Utilizing properties of the mutant reacting with the substrate via the binding therebetween, the mutant may be used as a signal protein for detecting the analyte. For example, by coupling or fusing the mutant with a protein capable of specifically recognizing the analyte by means of chemical coupling or fusion protein forming, when the mutant, the analyte, and the substrate are present in the same system, the protein specifically recognizing the analyte would bind to the analyte, and the mutant thereamong would catalyze the substrate, to perform the self-luminescence. The intensity of the bioluminescence released during the catalytic process of the mutant on the substrate may be measured with a microplate reader for chemiluminescence, and this intensity of the bioluminescence reflects the activity of the mutant, and the content of the analyte may be determined by such an activity level. It should be noted that before the binding of the substrate with the mutant, non-specific binding proteins in the system need to be removed, so as to eliminate the interference of other factors on the results. Therefore, the mutant may be used to prepare the kit to accurately detect a content of analyte.

According to specific embodiments of the present disclosure, the use as described above may further include at least one of the following additional technical features.

According to specific embodiments of the present disclosure, the content of the analyte is determined based on a fluorescence intensity change caused by the mutant before and after introducing the substrate for Gaussia luciferase or analog thereof.

According to specific embodiments of the present disclosure, the complex further includes a substrate for Gaussia luciferase or analog thereof.

According to specific embodiments of the present disclosure, the kit further includes a specific recognition protein for the analyte.

According to specific embodiments of the present disclosure, the substrate includes coelenterazine, fluoro-coelenterazine or coelenterazine derivatives. According to specific embodiments of the present disclosure, the substrate is not particularly limited, and any substance that chemically reacts with the mutant is included in the scope, which is not limited to chemiluminescence reaction, and the person skilled in the art could select different substrates according to the experimental needs.

According to some specific embodiments of the present disclosure, the coelenterazine derivative includes a coelenterazine derivative ZS2 or a coelenterazine derivative ZS26.

In yet another aspect of the present disclosure, there is provided in embodiments a kit for detecting a content of analyte, including the mutant described above, where a specific recognition protein of the analyte allows to form a complex with the mutant. Utilizing properties of the mutant reacting with the substrate via the binding therebetween, the mutant may be used as a signal protein for detecting the analyte. For example, a protein capable of specifically recognizing the analyte, by means of chemical coupling or fusion protein forming, may be coupled with or fused on the mutant, and the protein specifically recognizing the analyte would bind to the analyte, and the mutant thereamong would catalyze the substrate to present the self-luminescence. The intensity of the bioluminescence released during the catalytic process of the mutant on the substrate may be measured with a microplate reader for chemiluminescence, and this intensity of the bioluminescence reflects the activity of the mutant, and the content of the analyte may be determined by such an activity level. Thus, the kit including the mutant may be used to detect a content of analyte accurately.

According to specific embodiments of the present disclosure, the kit described above may further include at least one of the following additional technical features.

According to specific embodiments of the present disclosure, a substrate for Gaussia luciferase or analog thereof is further included in the kit.

According to specific embodiments of the present disclosure, the substrate includes coelenterazine, fluoro-coelenterazine or coelenterazine derivatives. According to specific embodiments of the present disclosure, the substrate is not particularly limited, and substances which chemically reacts with the mutant is included in the scope, which is not limited to chemiluminescence reaction, and the person skilled in the art could select different substrates according to the experimental needs.

According to specific embodiments of the present disclosure, the kit further includes a specific recognition protein for the analyte.

### Method

In one aspect, the present disclosure provides in embodiments a method of preparing a mutant, including: i) constructing an expression vector as described above; and ii) introducing the expression vector into a host cell to obtain the mutant. By the method according to specific embodiments of the present disclosure the mutant may be obtained simply and efficiently.

In another aspect, the present disclosure provides in embodiments a method for detecting a content of analyte, including: i) rendering a specific recognition protein of the analyte and the mutant as described above to form a complex, ii) contacting the analyte with the complex, iii) introducing a substrate for Gaussia luciferase or analog thereof to this system, and iv) determining a content of the analyte based on a fluorescence intensity change caused by the mutant before and after introducing the substrate for Gaussia luciferase or analog thereof. Utilizing properties of the mutant reacting with the substrate via the binding therebetween, a protein specifically recognizing the analyte may be coupled with or fused to the mutant by means of chemical coupling or fusion protein forming, which may specifically bind with the analyte, and the mutant therein has the ability to catalyze the luminescence of the substrate for Gaussia luciferase or analog thereof. When the fused or coupled protein is combined with the analyte, with removing non-specific binding proteins in the system, the substrate for Gaussia luciferase or analog thereof is introduced into the system, to measure, by a microplate reader for chemiluminescent, the bioluminescence released during the luminescence process of the substrate catalyzed by the mutant. As the bioluminescence measured reflects the combination of the mutant and the substrate, the content of the analyte may be determined by such an intensity. In addition, the mutant has a strong catalytic activity on substrates such as coelenterazine, fluoro-coelenterazine and coelenterazine derivatives, and has a wider substrate spectrum or higher substrate selectivity specificity and significantly enhanced luminescence brightness therein compared with the existing Gaussia luciferase, and the method according to the embodiment of the present disclosure can therefore detect a content of analyte more accurately.

According to specific embodiments of the present disclosure, the method described above may further include at least one of the following additional technical features.

According to specific embodiments of the present disclosure, the substrate for Gaussia luciferase or analog thereof includes coelenterazine, fluoro-coelenterazine or coelenterazine derivatives.

According to specific embodiments of the present disclosure, the coelenterazine derivative includes a coelenterazine derivative ZS2 or a coelenterazine derivative ZS26.

In yet anotheraspect, the present disclosure provides in embodiments a method for screening a substrate for Gaussia luciferase, including: i) contacting the mutant as described above with a substrate to be screened, and ii) determining whether the substrate to be screened is a target substrate or not based on a mixture in step i) whether emitting a chemical light signal or not.

According to specific embodiments of the present disclosure, the method described above may further include at least one of the following additional technical features.

According to specific embodiments of the present disclosure, the mixture in step i) emitting a chemical light signal indicates that the substrate to be screened is the target substrate.

The present disclosure is described below with reference to specific Examples, which are merely descriptive and do not limit the present disclosure in any way.

### Example 1 Design and construction of a prokaryotic expression plasmid for wild-type Gaussia luciferase

In this Example, the wild-type Gaussia luciferase with or without a signal peptide was constructed to determine the effect of the signal peptide on wild-type Gaussia luciferase by comparison.

The wild-type Gaussia luciferase with the signal peptide has a nucleic acid sequence as shown in SEQ ID NO: 1, and an amino acid sequence encoded by which is shown in SEQ ID NO: 2, containing an amino acid sequence of the signal peptide from position 1 to 17 (shown in bold), while the wild-type Gaussia luciferase without the signal peptide has an amino acid sequence as shown in SEQ ID NO: 3. A nucleic acid sequence of a further Gaussia luciferase protein with the signal peptide was synthesized with gene synthesis technology as shown in SEQ ID NO: 4, which was fused with a six-histidine (6 x His) tag at its C terminal for protein purification, with digestion sites of *BamHI* and *EcoRI* on its both ends. A nucleic acid sequence of another Gaussia luciferase protein without the signal peptide was synthesized by gene synthesis technology as shown in SEQ ID NO: 5, which is fused with a six-histidine (6 x His) tag at its C terminal for protein purification, with digestion sites of *NdeI* and *EcoRI* on its both ends.

ATGGGAGTGAAAGTTCTTTTTGCCCTTATTTGTATTGCTGTGGCCGAGGCCAAAC
CAACTGAAAACAATGAAGATTTCAACATTGTAGCTGTAGCTAGCAACTTTGCTACAAC

GGATCTCGATGCTGACCGTGGTAAATTGCCCGGAAAAAAATTACCACTTGAGGTACTC
AAAGAAATGGAAGCCAATGCTAGGAAAGCTGGCTGCACTAGGGGATGTCTGATATGC
CTGTCACACATCAAGTGTACACCCAAAATGAAGAAGTTTATCCCAGGAAGATGCCAC
ACCTATGAAGGAGACAAAGAAAGTGCACAGGGAGGAATAGGAGAGGCTATTGTTGAC
ATTCCTGAAATTCCTGGGTTTAAGGATTTGGAACCCATGGAACAATTCATTGCACAAG
TTGACCTATGTGTAGACTGCACAACTGGATGCCTCAAAGGTCTTGCCAATGTGCAATG
TTCTGATTTACTCAAGAAATGGCTGCCACAAAGATGTGCAACTTTTGCTAGCAAAATT
CAAGGCCAAGTGGACAAAATAAAGGGTGCCGGTGGTGAT (SEQ ID NO: 1)

MGVKVLFALICIAVAEAKPTENNEDFNIVAVASNFATTDLDADRGKLPGKKLPLEVL
KEMEANARKAGCTRGCLICLSHIKCTPKMKKFIPGRCHTYEGDKESAQGGIGEAIVDIPEI
PGFKDLEPMEQFIAQVDLCVDCTTGCLKGLANVQCSDLLKKWLPQRCATFASKIQGQVD
KIKGAGGD (SEQ ID NO: 2)

KPTENNEDFNIVAVASNFATTDLDADRGKLPGKKLPLEVLKEMEANARKAGCTRGC
LICLSHIKCTPKMKKFIPGRCHTYEGDKESAQGGIGEAIVDIPEIPGFKDLEPMEQFIAQVD
LCVDCTTGCLKGLANVQCSDLLKKWLPQRCATFASKIQGQVDKIKGAGGD (SEQ ID NO:
3)

ggatccaagcttgccgccaccATGGGAGTGAAAGTTCTTTTTGCCCTTATTTGTATTGCTGTG
GCCGAGGCCAAACCAACTGAAAACAATGAAGATTTCAACATTGTAGCTGTAGCTAGC
AACTTTGCTACAACGGATCTCGATGCTGACCGTGGTAAATTGCCCGGAAAAAAATTAC
CACTTGAGGTACTCAAAGAAATGGAAGCCAATGCTAGGAAAGCTGGCTGCACTAGGG
GATGTCTGATATGCCTGTCACACATCAAGTGTACACCCAAAATGAAGAAGTTTATCCC
AGGAAGATGCCACACCTATGAAGGAGACAAAGAAAGTGCACAGGGAGGAATAGGAG
AGGCTATTGTTGACATTCCTGAAATTCCTGGGTTTAAGGATTTGGAACCCATGGAACA
ATTCATTGCACAAGTTGACCTATGTGTAGACTGCACAACTGGATGCCTCAAAGGTCTT
GCCAATGTGCAATGTTCTGATTTACTCAAGAAATGGCTGCCACAAAGATGTGCAACTT
TTGCTAGCAAAATTCAAGGCCAAGTGGACAAAATAAAGGGTGCCGGTGGTGATggtggc
agtgagaacctgtacttccagagcggccaccaccaccaccatcacggctcctgatgagaattc (SEQ ID NO: 4)

catatgAAACCAACTGAAAACAATGAAGATTTCAACATTGTAGCTGTAGCTAGCAAC
TTTGCTACAACGGATCTCGATGCTGACCGTGGTAAATTGCCCGGAAAAAATTACCAC
TTGAGGTACTCAAAGAAATGGAAGCCAATGCTAGGAAAGCTGGCTGCACTAGGGGAT
GTCTGATATGCCTGTCACACATCAAGTGTACACCCAAAATGAAGAAGTTTATCCCAGG
AAGATGCCACACCTATGAAGGAGACAAAGAAAGTGCACAGGGAGGAATAGGAGAGG
CTATTGTTGACATTCCTGAAATTCCTGGGTTTAAGGATTTGGAACCCATGGAACAATTC
ATTGCACAAGTTGACCTATGTGTAGACTGCACAACTGGATGCCTCAAAGGTCTTGCCA

ATGTGCAATGTTCTGATTTACTCAAGAAATGGCTGCCACAAAGATGTGCAACTTTTGC
TAGCAAAATTCAAGGCCAAGTGGACAAAATAAAGGGTGCCGGTGGTGATggtggcagtgag
aacctgtacttccagagcggccaccaccaccaccatcacggctcctgataggaatt (SEQ ID NO: 5)

The synthesized wild-type Gaussia luciferase with (Glue WT) and without the signal peptide (no sp Glue) were individually dissolved in deionized water and diluted to 10 ng/µL as template DNA. With KOD FX neo enzyme (Toyo Textile, KFX-201S) a PCR system was prepared and a PCR was performed according to the instructions of the enzyme, to prepare insert fragments.

Primer sequences used for the PCR of wild-type Gaussia luciferase with the signal peptide (Glue WT) are shown in Table 1, the reaction system of which is shown in Table 2, and reaction conditions are shown in Table 3.

**Table 1**

| Name | Sequence (5'-3') |
|---|---|
| Upstream primer: Primer insert-F | gacagcaaatgggtcgcggatccaagcttgc (SEQ ID NO: 6) |
| Downstream primer: Primer insert-R | cgaattctcatcaggagccgtgatg (SEQ ID NO: 7) |

**Table 2**

| Components | Volume (µL) |
|---|---|
| 2x PCR buffer | 25 |
| 2 mM dNTPs | 10 |
| 10 µM Primer insert-F | 1.5 |
| 10 µM Primer insert-R | 1.5 |
| Template DNA (10 ng/µL) | 2.5 |
| KOD FX Neo | 1 |
| PCR grade water | 8.5 |
| Total volume | 50 |

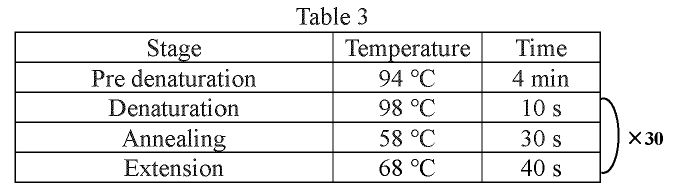

Dpnl enzyme (NEB, R0176S), taken for 0.5 µL, was added into the reaction system and incubated at 37 °C for 3 hours to digest the template, followed by recovering products with a length of 662 bp via gel as the insert fragments.

Taken pET28a vector as template, such a vector was linearized by PCR to facilitate recombination with the insert fragment. With KOD FX neo enzyme, a PCR system was prepared and a PCR was conducted according to the instructions of the enzyme, where PCR primers are shown in Table 4, the reaction system of which is shown in Table 5, and reaction conditions are shown in Table 6.

**Table 4**

| Name | Sequence (5'-3') |
|---|---|
| Upstream primer: Primer vector-F | catcacggctcctgatgagaattcg(SEQ ID NO: 8) |
| Downstream primer: Primer vector-R | gcaagcttggatccgcgacccatttgctgtc(SEQ ID NO: 9) |

**Table 5**

| Components | Volume (µL) |
|---|---|
| 2x KOD FX Neo PCR Buffer | 25 |
| 2 mM dNTPs enzyme free water | 10 |
| 10 µM Primer vector-F | 1.5 |
| 10 µM Primer vector-R | 1.5 |
| pET28a (10 ng/µL) | 2.5 |
| KOD FX Neo enzyme | 1 |
| PCR grade water | 8.5 |
| Total | 50 |

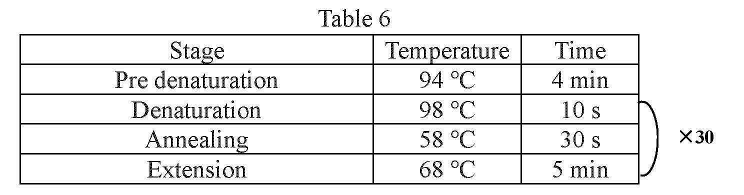

Dpnl enzyme, taken for 0.5 µL, was added to the reaction system and incubated at 37 °C for 3 hours to digest the template, followed by recovering products with a length of 5,318 bp via gel as the linearized vector.

With In-Fusion Cloning kit (TAKARA, Z9648N) the insert fragment and the linearized vector were incubated at 50 °C for 15 minutes for recombination, based on a reaction system shown in Table 7.

**Table 7**

| Components | Volume (µL) |
|---|---|
| insert (10 ng/µL) | 5 |
| vector (10 ng/µL) | 3 |
| Premix | 2 |
| Total volume | 10 |

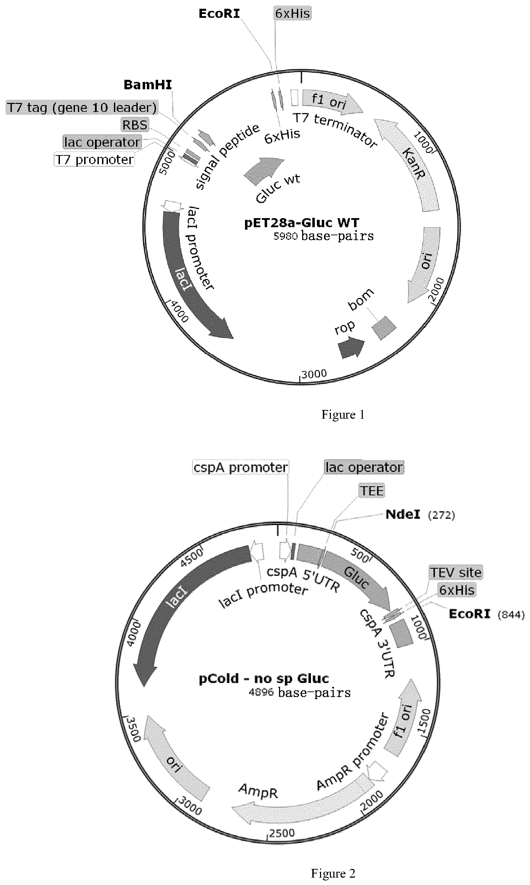

The above reaction products, taken for 2.5 µL, were transformed into DH5a competent cells, and coated on plate medium with kanamycin at a final concentration of 100 µg/mL. Single colonies were picked from the plates the next day to extract plasmids therein for sequencing, ensuring the target fragment being correctly inserted into the vector, and thus the obtained plasmids were the wild-type Gaussia luciferase with the signal peptide (pET28a-Gluc WT). The obtained plasmids were verified, and the specific results are shown in Figure 1, which was in line with the experimental expectations.

Primer sequences used for the PCR of wild-type without the signal peptide (no Gluc WT) are shown in Table 8, a reaction system of which is shown in Table 9, and reaction conditions are shown in Table 10.

**Table 8**

| Name | Sequence (5'-3') |
|---|---|
| Upstream primer: Primer insert-F | ccatgaatcacaaagtgcatatgaaaccaactgaaaacaatg(SEQ ID NO: 10) |
| Downstream primer: Primer insert-R | gcttttaagcagagattacctagaattcctatcaggagccgtg(SEQ ID NO: 11) |

**Table 9**

| Components | Volume (µL) |
|---|---|
| 2x PCR buffer for KOD FX Neo | 25 |
| 2 mM dNTPs | 10 |
| 10 µM Primer insert-F | 1.5 |
| 10 µM Primer insert-R | 1.5 |
| Template DNA (10 ng/µL) | 2.5 |
| KOD FX Neo | 1 |
| PCR grade water | 8.5 |
| Total volume | 50 |

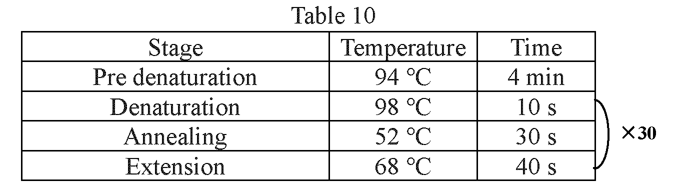

Dpnl enzyme, taken for 0.5 µL, was added into the reaction system and incubated at 37 °C for 3 hours to digest the template, followed by recovering products with a length of of 618 bp via gel as the insert fragments.

Taken pColdI vector, as template, such a vector was linearized by PCR to facilitate recombination with the insert fragment. With KOD FX neo enzyme, a PCR system was prepared and a PCR was conducted according to the instructions of the enzyme, where PCR primers are shown in Table 11, the reaction system of which is shown in Table 12, and reaction conditions are shown in Table 13.

**Table 11**

| Name | Sequence (5'-3') |
|---|---|
| Upstream primers | cacggctcctgataggaattctaggtaatctctgcttaaaagc(SEQ ID NO: 12) |
| Downstream primers | cattgttttcagttggtttcatatgcactttgtgattcatgg(SEQ ID NO: 13) |

**Table 12**

| Components | Volume (µL) |
|---|---|
| 2x KOD FX Neo PCR Buffer | 25 |
| 2 mM dNTPs | 10 |
| 10 µM Primer vector-F | 1.5 |
| 10 µM Primer vector-R | 1.5 |
| pColdI (10 ng/µL) | 2.5 |
| KOD FX Neo | 1 |
| PCR grade water | 8.5 |
| Total volume | 50 |

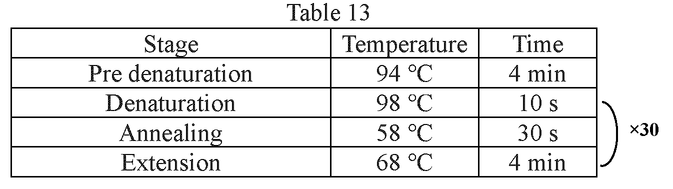

Dpnl enzyme, taken for 0.5 µL, was added into the reaction system and incubated at 37 °C for 3 hours to digest the template, followed by recovering products with a length of about 4,278 bp via gel as the linearized vectors. With In-Fusion Cloning kit (Takara) the insert fragment and the linearized vector were incubated at 50 °C for 15 minutes for recombination, based on a reaction system shown in Table 14.

**Table 14**

| Components | Volume (µL) |
|---|---|
| insert (10 ng/µL) | 5 |
| vector (10 ng/µL) | 3 |
| Premix | 2 |
| Total volume | 10 |

The above reaction products, taken for 2.5 µL, were transformed into DH5a competent cells, and coated on plate medium with ampicillin at a final concentration of 100 µg/mL. Single colonies were picked from the plates the next day to extract plasmids therein for sequencing, ensuring the target fragment being correctly inserted into the vector, and thus the obtained plasmids were the wild-type Gaussia luciferase without the signal peptide (pCold-no sp Glue). The obtained plasmids were verified, and the specific results are shown in Figure 2, which was in line with the experimental expectations.

### Example 2 Prokaryotic expression of Gaussia luciferase and their activity assay at bacteria solution level

In this example, the prokaryotic expression of the constructed Gaussia luciferase with and without the signal peptide, and the activity thereof at bacteria solution level were detected. The specific experimental operations were as follows.

The expression plasmids pET28a-Gluc wt and pCold-no sp Glue obtained in Example 1 were individually transformed into BL21 (DE3) competent cells or OrigamiB (DE3) chemically competent cells (WEIDI BIO, EC1020S) and applied to plate medium. Then single colonies were picked from the plate for culture overnight at 37 °C. The cultured clones were diluted at the ratio of 1:100 the next day, and transferred into 3 mL of fresh LB medium with ampicillin at 100 µg/mL, shaked at 37 °C, 200 rpm until OD600 ≈ 0.5-0.6, followed by cooling on ice for 1 hour. IPTG was added at a final concentration of 1 mM as inducer for induction overnight at 16 °C.

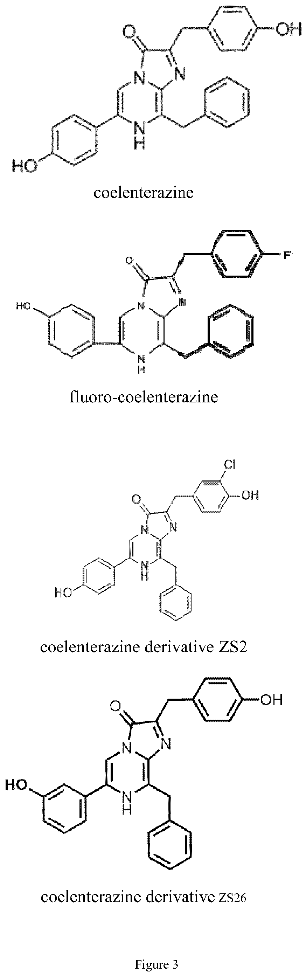

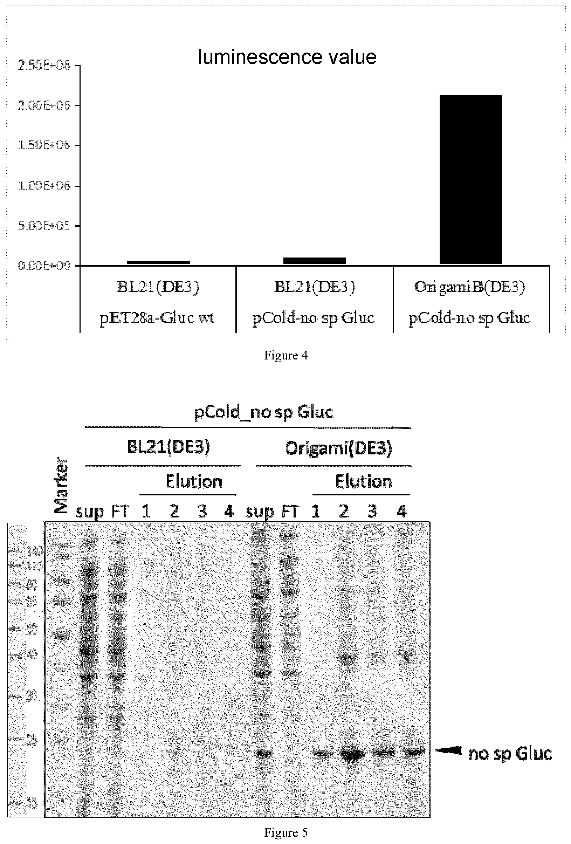

The induced bacteria solution, taken for 100 µL, was moved into a black 96-well plate, then added with 100 µL of a substrate CTZ (purchased from BIOLAB) at a final concentration of 100 µM. The structures of coelenterazine, fluoro-coelenterazine, and coelenterazine derivative ZS2 are shown in Figure 3. The luminescence intensity was read with a self-luminescence module of a microplate reader. The activity assay results of Gaussia luciferase at bacteria solution level are shown in Figure 4. Among them, OrigamiB (DE3) competent cells with the plasmid pCold-no sp Glue showed the highest luminescence value, indicating higher activity level of Gaussia luciferase without the signal peptide in bacteria solution. Accordingly, the subsequent experiments were conducted with the Gaussia luciferase without the signal peptide.

### Example 3 Prokaryotic expression Gaussia luciferase and protein purification thereof

The expression plasmids pCold-no sp Gluc same as the one used in Examples 1 and 2 were transformed into BL21 (DE3) competent cells or OrigamiB (DE3) chemically competent cells (WEIDI BIO, EC1020S) and applied to plate medium. Then single colonies were picked from the plate for culture overnight at 37 °C. The cultured clones were diluted at the ratio of 1:100 the next day, and transferred into 300 mL of fresh LB medium with kanamycin or ampicillin at 100 µg/mL, shaked at 37 °C, 200 rpm until OD600 ≈ 0.5-0.6, followed by cooling on ice for 1 hour. IPTG was added at a final concentration of 1 mM as inducer for induction overnight at 16 °C.

The induced bacteria solution was subject to centrifugation at 8,000 rpm/min for 10 min to collect precipitates, which were then added with 30 mL of binding buffer (50 mM Tris, pH 8.0, 250 mM NaCl) and 300 µL of lysozyme for lysis on ice for 30 min, and treated with ultrasonic sonication (2 s on, 3 s off, 60% power) for 30 min, followed by centrifugation at 4 °C and 12,000 rpm for 30 min to separate the supernatant (cell lysate) and precipitates.

2 ML of HisTrap FF packing were loaded into a manual column (purchased from Sangon Biotech, # F506607-0001, affinity chromatography column, unloaded), and flushed and balanced with 30 mL of binding buffer. Then about 30 mL of the filtered cell lysate were added to the column above, washed for 10 times (10 mL/time) with washing solution (50 mM Tris, pH 8.0, 250 mM NaCl, 10 mM imidazole) and then eluted with 500 µL of eluent (50 mM Tris, pH 8.0, 250 mM NaCl, 300 mM imidazole) for 4-5 times. The eluted protein was collected and detected with 12% SDS-PAGE. The purification results are shown in Figure 5, which indicates that a relatively pure no sp Gluc protein was obtained from components of the elution 1 of Origami (DE3).

### Example 4 Design and construction of plasmids for prokaryotic expression of a Gaussia luciferase mutant library

Through gene synthesis technology, oligo pools of the Gaussia luciferase mutant randomly mutated at the specified position of SEQ ID NO: 4 were synthesized. The mutant library 1 (L1) was randomly mutated at positions F26, N27, V29, A30, S33, A36, T37, and L40 of the amino acid sequence of SEQ ID NO: 4, the mutant library 2 (L2) was randomly mutated at positions K66, H79, T84, E102, E110, D123, L124 and V138 of the amino acid sequence of SEQ ID NO: 4, and the mutant library 3 (L3) was randomly mutated at Q152, Q163, T167, S170, G174, Q175, K178, A182 and G183 of the amino acid sequence of SEQ ID NO: 4.

The synthesized mutant libraries L1, L2 and L3 were individually dissolved in deionized water and diluted to 10 ng/µL as template DNA. With KOD FX neo enzyme, a PCR system was prepared and a PCR was performed according to the instructions of the enzyme, to prepare insert fragments. The PCR system, PCR conditions, and In-fusion recombination method were the same as the preparation method of Gaussia luciferase without the signal peptide (no sp Glue) in Example 1.

The above recombinant products, taken for 3.5 µL, were transformed into EPI300 competent cells (Lucigen, EC300110), and coated on plate medium containing ampicillin at a final concentration of 100 µg/mL, and all single colonies were collected from L1, L2, and L3 plates the next day, to extract plasmids therein. The plasmids were conducted with sequencing to ensure the target fragment being correctly inserted into the vector, and the verified plasmids as follows were expression plasmids of these mutant libraries respectively: pCold-no sp Gluc L1, pCold-no sp Glue L2, and pCold-no sp Gluc L3.

### Example 5 Prokaryotic expression and protein purification of Gaussia luciferase mutant library

The mutant libraries pCold-no sp Gluc L1, pCold-no sp Gluc L2 and pCold-no sp Glue L3 obtained in Example 4 were individually transformed into OrigamiB (DE3) chemically competent cells (WEIDI BIO, EC1020S) and coated on plates containing ampicillin. Single colonies were picked from respective plates and incubated at 37 °C overnight. The cultured clones were diluted at the ratio of 1:100 the next day, and transferred into 3 mL of fresh LB medium with ampicillin at 100 µg/mL, shaked at 37 °C, 200 rpm until OD600 ≈ 0.5-0.6, followed by cooling on ice for 1 hour. IPTG was added at a final concentration of 1 mM as inducer for for induction overnight at 16 °C.

The induced bacteria solution was subject to centrifugation at 8,000 rpm/min for 10 min to collect precipitates, which were then added with 600 µL of binding buffer (50 mM Tris, pH 8.0, 250 mM NaCl) and 6 µL of lysozyme for lysis on ice for 30 min, and then treated with ultrasonic sonication (2 s on, 3 s off, 60% power) for 10 min, followed by centrifugation at 4 °C and 12,000 rpm for 30 min to separate the supernatant (cell lysate) and precipitates.

HisTrap FF packing, taken for 50 µL, was loaded into a manual column (purchased from Sangon Biotech, # F506607-0001, affinity chromatography column, unloaded), and flushed and balanced with 3 mL of binding buffer. Then about 3 mL of the filtered cell lysate were added to the column above, washed for 10 times (3 mL/time) with washing solution (50 mM Tris, pH 8.0, 250 mM NaCl, 10 mM imidazole) and eluted with 100 µL of eluent (50 mM Tris, pH 8.0, 250 mM NaCl, 300 mM imidazole), to collect the eluted proteins.

The protein eluted from Ni column was dialyzed overnight at 4 °C with dialysis buffer (25 mM Tris, pH 8.0, 250 mM NaCl). The protein concentration and purity distribution were determined by BCA quantitative assay and SDS-PAGE assay, respectively.

### Example 6 Activity assay on advantageous mutants of Gaussia luciferase

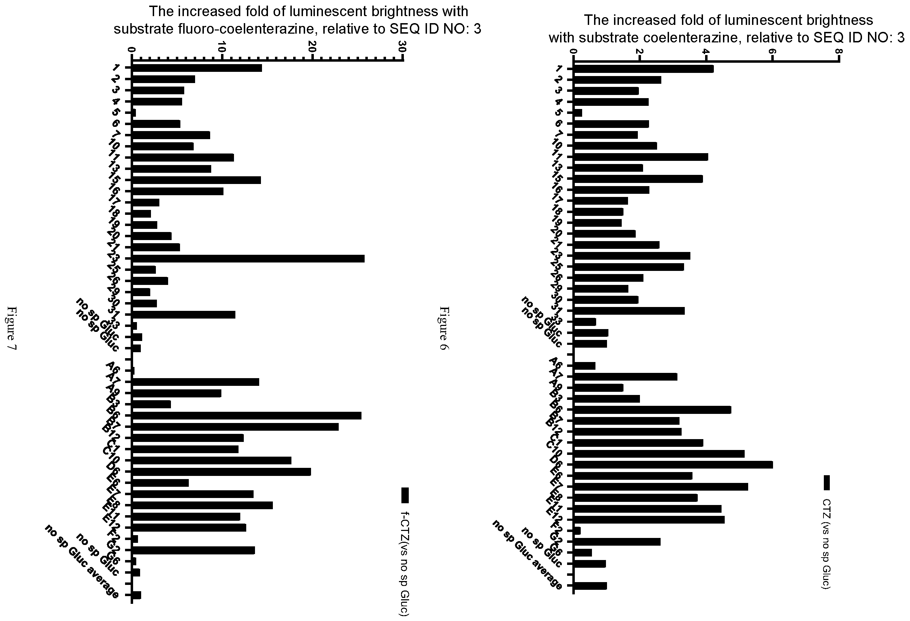

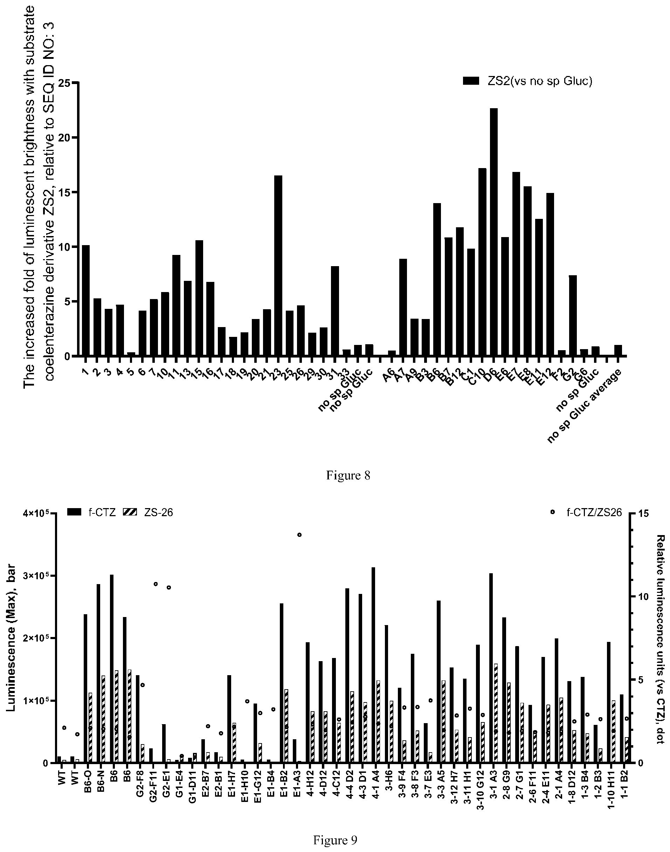

In this Example, the protein concentration was determined with a BCA quantitative kit (Thermo Scientific^{™} Pierce^{™} BCA Protein Assay Kit) accurately. The purified luciferase obtained in Example 5 was diluted with diluent (50 mM Tris-HCl, pH 8.0, 100 mM NaCl, 0.1% (v/v) Tween-20) to 1 µg/mL. The diluted luciferase, taken for 10 µL, was moved into a black 96-well plate, then added with 90 µL of the substrates coelenterazine, fluoro-coelenterazine or coelenterazine derivative ZS2 (purchased from Biolab) which was diluted into 100 µM with the same diluent, and the luminescence intensity of the plate was read with a self-luminescence module of a microplate reader. The mutation site of the mutant, the measured luminescence intensity, the ratio of luminescence intensities of the advantageous mutants to the wild-type Gaussia luciferase without the signal peptide having the sequence shown in SEQ ID NO: 3 are shown in Table 15. The results of catalytic activity assay of the advantageous mutants relative to the wild-type Gaussia luciferase without the signal peptide having the sequence shown in SEQ ID NO: 3 on substrate coelenterazine are shown in Figure 6. Among them, the following eight mutation combinations had significantly improved catalytic activities on coelenterazine, identifiers of which were D6 (H79K, P84A, E102D, S103T, A104G, L124M, V138E), E7 (K66R, H79K, P84L, S103T, A104G, V138E), C10 (K66R, H79K, P84A, E102A, S103T, A104G, L124M, V138D), B6 (H79K, P84L, E102S, S103T, A104G, L124M, V138E), E12 (K66N, H79K, E102A, S103T, A104G, V138E), 1(K66R, H79K, P84A, E102S, A104G, L124M, V138E), 11(H79K, P84A, E102D, A104G, E110A, L124M), and E11(P84A, E102K, A104G, E110A, L124M). The results of catalytic activity assay of the advantageous mutants relative to the wild-type Gaussia luciferase without the signal peptide having the sequence shown in SEQ ID NO: 3 on substrate fluoro-coelenterazine are shown in Figure 7. Among them, the following 11 mutation combinations had significantly improved catalytic activities on fluoro-coelenterazine, identifiers of which were 23 (K66N, H79K, P84L, E102S, S103T, A104G, E110P, L124M, V138D), B7 (K66R, P84A, E102A, S103T, A104G, E110P, L124M, V138E), A9 (K66R, P84L, K88R, E102A, S103T, A104G, L124M), B6 (H79K, P84L, E102S, S103T, A104G, L124M, V138E), G2 (K66N, P84K, S103T, A104G, E110G, D123N, L124M, V138E), C10 (K66R, H79K, P84A, E102A, S103T, A104G, L124M, V138D), D6(H79K, P84A, E102D, S103T, A104G, L124M, V138E), E8(P84K, E102A, S103T, A104G, E110P),1(K66R, H79K, P84A, E102S, A104G, L124M, V138E), 15(K66N, H79K, P84A, E102D, S103T, A104G, V138D), and A7(K66N, P84V, E102N, S103T, A104G, E110A, L124M, V138D). The results of catalytic activity assay of the advantageous mutants relative to the wild-type Gaussia luciferase without the signal peptide having the sequence shown in SEQ ID NO: 3 on substrate coelenterazine derivative ZS2 are shown in Figure 8. Among them, the following 7 mutation combinations had significantly improved catalytic activities on the coelenterazine derivative ZS2, identifiers of which were D6 (H79K, P84A, E102D, S103T, A104G, L124M, V138E), 23 (K66N, H79K, P84L, E102S, S103T, A104G, E110P, L124M, V138D), C10 (K66R, H79K, P84A, E102A, S103T, A104G, L124M, V138D), E7 (K66R, H79K, P84L, S103T, A104G, V138E), E8(P84K, E102A, S103T, A104G, E110P), B6 (H79K, P84L, E102S, S103T, A104G, L124M, V138E), and E12 (K66N, H79K, E102A, S103T, A104G, V138E).

**Table 15**

| No. | Mutations in mutant compared with SEQ ID NO: 3 | CTZ | | f-CTZ | | ZS2 | |
|---|---|---|---|---|---|---|---|
| | | Measured luminescence intensity | Ratio of luminescence intensities of advantageous mutant to wild-type Gaussia luciferase without signal peptide | Measured luminescence intensity | Ratio of luminescence intensities of advantageous mutant to wild-type Gaussia luciferase without signal peptide | Measured luminesce -ence intensity | Ratio of luminescence intensities of advantageous mutant to wild-type Gaussia luciferase without signal peptide |
| 1 | K66R, H79K, P84A, E102S, A104G, L124M, V138E | 1120037 | 4.22 | 91364 | 14.39 | 207316 | 10.12 |
| 2 | K66N, P84A, E102S, S103T, E110A, L124M, V138E | 700056 | 2.64 | 44352 | 6.98 | 107651 | 5.25 |
| 3 | K66R, P84L, E102S, S103T, L124M, V138E | 521015 | 1.96 | 36666 | 5.77 | 87830 | 4.29 |
| 4 | K66N, P84A, E102A, S103T, L124M | 599544 | 2.26 | 35315 | 5.56 | 95582 | 4.67 |
| 5 | K66S, P84A, E102D, S103T, E110P,V138D | 66456 | 0.25 | 2810 | 0.44 | 7246 | 0.35 |
| 6 | K66N, P84A, E102D, S103T, E110P,V138D | 603572 | 2.27 | 34060 | 5.36 | 84427 | 4.12 |
| 7 | K66N, P84K, S103T, A104G, E110A, L124M | 511385 | 1.93 | 54844 | 8.64 | 105898 | 5.17 |
| 10 | P84L, E102A, S103T, E110A, L124M, V138D | 666644 | 2.51 | 43498 | 6.85 | 119487 | 5.83 |
| 11 | H79K, P84A, E102D, A104G, E110A, L124M | 1074356 | 4.05 | 71828 | 11.31 | 188829 | 9.22 |
| 13 | K66R, P84K, E102D, A104G, E110P, V138D | 555457 | 2.09 | 55707 | 8.77 | 140317 | 6.85 |
| 15 | K66N, H79K, P84A, E102D, S103T, A104G, V138D | 1032953 | 3.89 | 90756 | 14.29 | 216796 | 10.58 |
| 16 | K66N, P84K, E102A, S103T, A104G, E110A, V138D | 605094 | 2.28 | 64185 | 10.11 | 138666 | 6.77 |
| 17 | P84A, E102A, S103T, E110A, V138D | 431743 | 1.63 | 19377 | 3.05 | 53306 | 2.6 |
| 18 | K66N, H79K, P84A, S103T, V138D | 398281 | 1.5 | 13274 | 2.09 | 35667 | 1.74 |
| 19 | E102S, S103T, L124M, V138D | 382878 | 1.44 | 17772 | 2.8 | 43493 | 2.12 |
| 20 | K66N, P84A, E102A, E110P, L124M, V138D | 493552 | 1.86 | 27784 | 4.38 | 68657 | 3.35 |
| 21 | H79K, P84L, E102S, S103T, E110P, L124M, V138D | 685655 | 2.58 | 33693 | 5.31 | 87416 | 4.27 |
| 23 | K66N, H79K, P84L, E102S, S103T, A104G, E110P, L124M, V138D | 931325 | 3.51 | 163397 | 25.73 | 338601 | 16.53 |
| 25 | H79K, P84L, E102D, S103I, A104G, L124M | 880672 | 3.32 | 16918 | 2.66 | 84409 | 4.12 |
| 26 | K66R, P84A, E102S, S103T, E110P, L124M | 556641 | 2.1 | 25375 | 4 | 94156 | 4.6 |
| 29 | K66N, P84A, E102S, E110P, L124M, V138E | 434906 | 1.64 | 12771 | 2.01 | 43192 | 2.11 |
| 30 | K66N, H79K, P84V, E102D, S103I, E110A, L124M, V138D | 518734 | 1.95 | 17545 | 2.76 | 52732 | 2.57 |
| 31 | H79K, E102K, S103T, A104G, L124M, V138D | 886619 | 3.34 | 72541 | 11.42 | 168160 | 8.21 |
| 33 | T167S S170V G174S K178N A182L | 177238 | 0.67 | 3526 | 0.56 | 12196 | 0.6 |
| - - | no sp Gluc | 276603 | 1.04 | 7198 | 1.13 | 20707 | 1.01 |
| - - | no sp Gluc | 266322 | 1 | 6191 | 0.97 | 22415 | 1.09 |
| | | | | | | | |
| A6 | F26S V29E A32V S33N T37E L40G | 173222 | 0.65 | 1660 | 0.26 | 10106 | 0.49 |
| A7 | K66N, P84V, E102N, S103T, A104G, E110A, L124M, V138D | 827926 | 3.12 | 89286 | 14.06 | 181477 | 8.86 |
| A9 | K66R, P84L, K88R, E102A, S103T, A104G, L124M | 399345 | 1.5 | 62747 | 9.88 | 69731 | 3.4 |
| B3 | | 528603 | 1.99 | 27441 | 4.32 | 68508 | 3.34 |
| B6 | H79K, P84L, E102S, S103T, A104G, L124M, V138E | 1258637 | 4.74 | 161276 | 25.4 | 286599 | 13.99 |
| B7 | K66R, P84A, E102A, S103T, A104G, E110P, L124M, V138E | 847026 | 3.19 | 145354 | 22.89 | 222206 | 10.85 |
| B12 | P84L, E102S, S103T, A104G, E110P, L124M | 862192 | 3.25 | 78627 | 12.38 | 241432 | 11.78 |
| C1 | K66N, P84K, E102A, A104G, D123N, L124M, V138D | 1036646 | 3.9 | 74868 | 11.79 | 200846 | 9.8 |
| C10 | K66R, H79K, P84A, E102A, S103T, A104G, L124M, V138D | 1366784 | 5.15 | 111998 | 17.64 | 351404 | 17.15 |
| D6 | H79K, P84A, E102D, S103T, A104G, L124M, V138E | 1593746 | 6 | 125777 | 19.81 | 464311 | 22.66 |
| E6 | P84K, E102A, S103T, E110P, D123N, L124M, V138D | 951799 | 3.58 | 40026 | 6.3 | 222988 | 10.88 |
| E7 | K66R, H79K, P84L, S103T, A104G, V138E | 1392905 | 5.25 | 85419 | 13.45 | 345303 | 16.85 |
| E8 | P84K, E102A, S103T, A104G, E110P | 989404 | 3.73 | 99075 | 15.6 | 318235 | 15.53 |
| E11 | P84A, E102K, A104G, E110A, L124M | 1181853 | 4.45 | 76131 | 11.99 | 257151 | 12.55 |
| E12 | K66N, H79K, E102A, S103T, A104G, V138E | 1206470 | 4.54 | 80368 | 12.66 | 305560 | 14.91 |
| F2 | | 56724 | 0.21 | 4289 | 0.68 | 11055 | 0.54 |
| G2 | K66N, P84K, S103T, A104G, E110G, D123N, L124M, V138E | 696744 | 2.62 | 86404 | 13.61 | 150805 | 7.36 |
| G6 | K66N, P84K, S103T, A104G, E110G, D123N, L124M, V138E | 143011 | 0.54 | 2957 | 0.47 | 12668 | 0.62 |
| - - | no sp Gluc | 253597 | 0.96 | 5662 | 0.89 | 18344 | 0.9 |
| | no sp Gluc average | 265507.3 | 1 | 6350.333 | 1 | 20488.67 | 1 |

**Table 16**

| Mutations in mutant compared with SEQ ID NO: 3 | Substrate | Activity of mutant relative to wild-type Gaussia luciferase without the signal peptide |
|---|---|---|
| A152H Q163S T167S S170V G174K K178T A182Y G183A | ZS2 | 0.750 |
| A152H Q163S T167S S170V G174K K178T A182Y G183A | CTZ | 1.005 |
| A17V H79K P84A E102D S103T E110P L124M | f-CTZ | 0.763 |
| A17V H79K P84A E102D S103T E110P L124M | CTZ | 0.198 |
| A17V H79K P84A E102D S103T E110P L124M | ZS2 | 0.082 |
| A30G V31I A32V S33G A36N T37D L40R | f-CTZ | 0.615 |
| A30G V31I A32V S33G A36N T37D L40R | CTZ | 0.138 |
| A30G V31I A32V S33G A36N T37D L40R | ZS2 | 0.116 |
| A42G Q152C T167G S170T G174N K178S A182Y G183A | CTZ | 2.527 |
| A42G Q152C T167G S170T G174N K178S A182Y G183A | f-CTZ | 0.520 |
| A42G Q152C T167G S170T G174N K178S A182Y G183A | ZS2 | 1.065 |
| A42G Q152H T167G S170T G174N K178S A182Y G183A | f-CTZ | 0.657 |
| A42G Q152H T167G S170T G174N K178S A182Y G183A | CTZ | 0.568 |
| A42G Q152H T167G S170T G174N K178S A182Y G183A | ZS2 | 0.001 |
| A42G Q152K S170S G174N Q175E K178S A182Y G183A | ZS2 | 1.751 |
| A42G Q152K S170S G174N Q175E K178S A182Y G183A | CTZ | 0.736 |
| de155-56EV Q152R Q163S S170V G174S Q175E A180Y G181A | ZS2 | 0.095 |
| de155-56EV Q152R Q163S S170V G174S Q175E A180Y G181A | CTZ | 0.098 |
| E102K S103T A104G E110G V138D | f-CTZ | 4.919 |
| E102K S103T A104G E110G V138D | CTZ | 0.106 |
| E102K S103T A104G E110G V138D | ZS2 | 0.065 |
| E102K S103T A104G E110G V138D I172 | f-CTZ | 2.719 |
| E102K S103T A104G E110G V138D I172 | CTZ | 0.622 |
| E102K S103T A104G E110G V138D I172 | ZS2 | 0.550 |
| E102K S103T E110A | ZS2 | 1.504 |
| E102K S103T E110A | CTZ | 0.864 |
| F26A N27D V29R A30G A32E S33G A36P L40A | ZS2 | 0.684 |
| F26A N27D V29R A30G A32E S33G A36P L40A | CTZ | 0.034 |
| F26C N27D A30D A32V S33R A36T | f-CTZ | 0.130 |
| F26C N27D A30D A32V S33R A36T | CTZ | 0.126 |
| F26C N27D A30D A32V S33R A36T | ZS2 | 0.166 |
| F26C N27D A30V V31L A32G S33I A36V T37M L40G | f-CTZ | 0.065 |
| F26C N27D A30V V31L A32G S33I A36V T37M L40G | CTZ | 0.054 |
| F26C N27D A30V V31L A32G S33I A36V T37M L40G | ZS2 | 0.086 |
| F26C N27D V29L A32V A36I T37A L40I | CTZ | 0.203 |
| F26C N27D V29L A32V A36I T37A L40I | f-CTZ | 0.141 |
| F26C N27D V29L A32V A36I T37A L40I | ZS2 | 0.166 |
| F26C V29A V31L A32D S33G A36V T33K L40S | f-CTZ | 0.074 |
| F26C V29A V31L A32D S33G A36V T33K L40S | CTZ | 0.072 |
| F26C V29A V31L A32D S33G A36V T33K L40S | ZS2 | 0.092 |
| F26C V29L A30D V31L A32V S33G L40I | ZS2 | 0.312 |
| F26C V29L A30D V31L A32V S33G L40I | CTZ | 0.000 |
| F26C V29Y S33G A36I T37E L40T | ZS2 | 1.131 |
| F26C V29Y S33G A36I T37E L40T | CTZ | 0.016 |
| F26D N26Y V29G A30V V31L S33D A36D T37H L40M | ZS2 | 0.176 |
| F26D N26Y V29G A30V V31L S33D A36D T37H L40M | CTZ | 0.011 |
| F26D N27D V29O A30D V31I S33R T37L | ZS2 | 0.213 |
| F26D N27D V29O A30D V31I S33R T37L | CTZ | 1.110 |
| F26D N27H V29K A30G A32G S33L T37L L40R | ZS2 | 0.381 |
| F26D N27H V29K A30G A32G S33L T37L L40R | CTZ | 0.037 |
| F26G N27D V29L A30V V31I A32G S33D A36W T37S | f-CTZ | 0.040 |
| F26G N27D V29L A30V V31I A32G S33D A36W T37S | CTZ | 0.038 |
| F26G N27D V29L A30V V31I A32G S33D A36W T37S | ZS2 | 0.027 |
| F26G N27Y A30D V31L A32V S33R A36M T37E L40G | ZS2 | 1.203 |
| F26G N27Y A30D V31L A32V S33R A36M T37E L40G | CTZ | 0.003 |
| F26G N27Y A30G V31L A32G S33L A36Y T37V L40E | f-CTZ | 0.025 |
| F26G N27Y A30G V31L A32G S33L A36Y T37V L40E | CTZ | 0.031 |
| F26G N27Y A30G V31L A32G S33L A36Y T37V L40E | ZS2 | 0.027 |
| F26GN27Y V29C A30V V31L S33G A36R T37A L40A | f-CTZ | 0.104 |
| F26GN27Y V29C A30V V31L S33G A36R T37A L40A | CTZ | 0.088 |
| F26GN27Y V29C A30V V31L S33G A36R T37A L40A | ZS2 | 0.080 |
| F26I V29F A30G A32E S33D A36N T37D L40T | ZS2 | 0.000 |
| F26I V29F A30G A32E S33D A36N T37D L40T | CTZ | 1.120 |
| F26I A30D A32V S33G A36D T37K L40V | ZS2 | 2.392 |
| F26I A30D A32V S33G A36D T37K L40V | CTZ | 0.636 |
| F26I N27D V29A A30D V31I S33K A36N T37K L40P | f-CTZ | 0.080 |
| F26I N27D V29A A30D V31I S33K A36N T37K L40P | CTZ | 0.016 |
| F26I N27D V29A A30D V31I S33K A36N T37K L40P | ZS2 | 0.026 |
| F26I N27D V29D A30G A32E S33G A36T L40S del117-119 D139H | ZS2 | 0.010 |
| F26I N27D V29D A30G A32E S33G A36T L40S del117-119 D139H | CTZ | 0.043 |
| F26I N27D V29S A30G V31L A32V S33D A36V T37E L40G | ZS2 | 2.094 |
| F26I N27D V29S A30G V31L A32V S33D A36V T37E L40G | CTZ | 0.788 |
| F26I N27H V29G A32G S33D A36G T37L L40I | ZS2 | 0.479 |
| F26I N27H V29G A32G S33D A36G T37L L40I | CTZ | 0.055 |
| F26I V29A A30D A32V S33K T37E L40A | CTZ | 1.470 |
| F26I V29A A30D A32V S33K T37E L40A | f-CTZ | 0.583 |
| F26I V29A A30D A32V S33K T37E L40A | ZS2 | 1.231 |
| F26L N27D A30D V31L A32V S33N A36N T37A L40S | ZS2 | 1.802 |
| F26L N27D A30D V31L A32V S33N A36N T37A L40S | CTZ | 0.799 |
| F26L N27D V29A V31L A32D S33N T37E L40T | ZS2 | 0.191 |
| F26L N27D V29A V31L A32D S33N T37E L40T | CTZ | 0.000 |
| F26L N27D V29D A30G S33N A36I T37D L40R | ZS2 | 1.989 |
| F26L N27D V29D A30G S33N A36I T37D L40R | CTZ | 0.726 |
| F26L N27D V29P A30G V31I A32G S33A A36T T37C L40E I112T | ZS2 | 0.395 |
| F26L N27D V29P A30G V31I A32G S33A A36T T37C L40E I112T | CTZ | 0.001 |
| F26L N27H V29C V31I S33V A36Q T37S L40G | f-CTZ | 0.095 |
| F26L N27H V29C V31I S33V A36Q T37S L40G | CTZ | 0.066 |
| F26L N27H V29C V31I S33V A36Q T37S L40G | ZS2 | 0.067 |
| F26L V29A V31I A32D S33R A36N T37E L40P | f-CTZ | 0.402 |
| F26L V29A V31I A32D S33R A36N T37E L40P | CTZ | 0.406 |
| F26L V29A V31I A32D S33R A36N T37E L40P | ZS2 | 0.385 |
| F26N V29S A30D S33D A36T T37A L40G | f-CTZ | 0.121 |
| F26N V29S A30D S33D A36T T37A L40G | CTZ | 0.098 |
| F26N V29S A30D S33D A36T T37A L40G | ZS2 | 0.106 |
| F26R N27D V29C A30G V31L A32D S33V A36V T37S | ZS2 | 0.128 |
| F26R N27D V29C A30G V31L A32D S33V A36V T37S | CTZ | 1.308 |
| F26R N27D V29D A30G A32D S33N T37A L40P | f-CTZ | 0.049 |
| F26R N27D V29D A30G A32D S33N T37A L40P | CTZ | 0.031 |
| F26R N27D V29D A30G A32D S33N T37A L40P | ZS2 | 0.031 |
| F26R N27D V29E A30G V31I A32D S33N A36N T37A L40R | f-CTZ | 0.067 |
| F26R N27D V29E A30G V31I A32D S33N A36N T37A L40R | CTZ | 0.053 |
| F26R N27D V29E A30G V31I A32D S33N A36N T37A L40R | ZS2 | 0.044 |
| F26R N27Y V29E A30G A32D S33R A36G T37M L40P | f-CTZ | 0.172 |
| F26R N27Y V29E A30G A32D S33R A36G T37M L40P | CTZ | 0.152 |
| F26R N27Y V29E A30G A32D S33R A36G T37M L40P | ZS2 | 0.147 |
| F26R V29D A30G A32D A36I T37A L40R | ZS2 | 2.460 |
| F26R V29D A30G A32D A36I T37A L40R | CTZ | 0.453 |
| F26R V29E A30G V31L A36D T37E L40S K159 | f-CTZ | 0.009 |
| F26R V29E A30G V31L A36D T37E L40S K159 | CTZ | 0.000 |
| F26R V29E A30G V31L A36D T37E L40S K159 | ZS2 | 0.001 |
| F26R V29F A30D V31L S33G T37D L40A | ZS2 | 1.781 |
| F26R V29F A30D V31L S33G T37D L40A | CTZ | 1.017 |
| F26R V29R A30V V31L S33G A36V T37R | CTZ | 0.423 |
| F26R V29R A30V V31L S33G A36V T37R | ZS2 | 0.115 |
| F26S N27Y V29C A32G S33E A36Q T37G L40T | f-CTZ | 0.143 |
| F26S N27Y V29C A32G S33E A36Q T37G L40T | CTZ | 0.119 |
| F26S N27Y V29C A32G S33E A36Q T37G L40T | ZS2 | 0.126 |
| F26S V29L A30G S33R T37K L40A | ZS2 | 0.787 |
| F26S V29L A30G S33R T37K L40A | CTZ | 1.067 |
| F26T N27D V29P A30G V31I A32G S33I A36I T37L L40I | f-CTZ | 0.210 |
| F26T N27D V29P A30G V31I A32G S33I A36I T37L L40I | CTZ | 0.231 |
| F26T N27D V29P A30G V31I A32G S33I A36I T37L L40I | ZS2 | 0.224 |
| F26T N27N V29E V31I A32G S33V A36R T37G L40G | ZS2 | 1.995 |
| F26T N27N V29E V31I A32G S33V A36R T37G L40G | CTZ | 0.940 |
| F26V A30V A30G S33L A36P T37P L40G | ZS2 | 0.365 |
| F26V A30V A30G S33L A36P T37P L40G | CTZ | 1.037 |
| F26V N27Y V29N A30D V31D D32L S33R A36R T37W L40Y K180R | f-CTZ | 0.065 |
| F26V N27Y V29N A30D V31D D32L S33R A36R T37W L40Y K180R | CTZ | 0.072 |
| F26V N27Y V29N A30D V31D D32L S33R A36R T37W L40Y K180R | ZS2 | 0.063 |
| F26Y N27Y V29A A30G S33V A36V T37S L40G | f-CTZ | 0.192 |
| F26Y N27Y V29A A30G S33V A36V T37S L40G | CTZ | 0.219 |
| F26Y N27Y V29A A30G S33V A36V T37S L40G | ZS2 | 0.177 |
| H79K P84A E102S S103I A104G E110P L124M | f-CTZ | 0.007 |
| H79K P84A E102S S103I A104G E110P L124M | CTZ | 0.000 |
| H79K P84A E102S S103I A104G E110P L124M | ZS2 | 0.001 |
| H79K P84K E102K S103I V138E | f-CTZ | 0.479 |
| H79K P84K E102K S103I V138E | CTZ | 0.750 |
| H79K P84K E102K S103I V138E | ZS2 | 0.452 |
| H79K P84L S103T A104G E110A | f-CTZ | 13.108 |
| H79K P84L S103T A104G E110A | CTZ | 1.921 |
| H79K P84L S103T A104G E110A | ZS2 | 0.064 |
| H79K P84L V138D | f-CTZ | 0.936 |
| H79K P84L V138D | CTZ | 0.094 |
| H79K P84L V138D | ZS2 | 0.068 |
| I10T C11S A13D A15I E16R A17S I18K | CTZ | 0.075 |
| I10T C11S A13D A151 E16R A17S I18K | f-CTZ | 0.044 |
| I10T C11S A13D A15I E16R A17S I18K | ZS2 | 0.059 |
| I10T C11S A13D A15I E16R A17S | f-CTZ | 0.086 |
| I10T C11S A13D A15I E16R A17S | CTZ | 0.071 |
| I10T C11S A13D A15I E16R A17S | ZS2 | 0.116 |
| K66N E101A S103T A104G E110G | f-CTZ | 3.673 |
| K66N E101A S103T A104G E110G | CTZ | 1.060 |
| K66N E101A S103T A104G E110G | ZS2 | 1.748 |
| K66N E102A S103T A104G E110G D123N L124M V138D K180 | f-CTZ | 6.874 |
| K66N E102A S103T A104G E110G D123N L124M V138D K180 | CTZ | 0.945 |
| K66N E102A S103T A104G E110G D123N L124M V138D K180 | ZS2 | 1.880 |
| K66N E102D S103T E110G L124M V138E | f-CTZ | 0.794 |
| K66N E102D S103T E110G L124M V138E | CTZ | 0.134 |
| K66N E102D S103T E110G L124M V138E | ZS2 | 0.471 |
| K66N H79K E102D E110P L124M | CTZ | 1.554 |
| K66N H79K E102D E110P L124M | f-CTZ | 0.404 |
| K66N H79K E102D E110P L124M | ZS2 | 0.751 |
| K66N H79K P84A E102E S103I D123N L124M V138E K159N I179* | f-CTZ | 0.009 |
| K66N H79K P84A E102E S103I D123N L124M V138E K159N I179* | CTZ | 0.000 |
| K66N H79K P84A E102E S103I D123N L124M V138E K159N I179* | ZS2 | 0.001 |
| K66N H79K P84K E102K S103I E110P L124M | f-CTZ | 1.728 |
| K66N H79K P84K E102K S103I E110P L124M | CTZ | 0.755 |
| K66N H79K P84K E102K S103I E110P L124M | ZS2 | 1.118 |
| K66N H79K P84L S103T E110A D123N L124M E128G V138E | ZS2 | 1.709 |
| K66N H79K P84L S103T E110A D123N L124M E128G V138E | CTZ | 1.111 |
| K66N P84A E102K S103I E110G L124M V138E | f-CTZ | 0.896 |
| K66N P84A E102K S103I E110G L124M V138E | CTZ | 0.654 |
| K66N P84A E102K S103I E110G L124M V138E | ZS2 | 0.620 |
| K66N P84L E102A S103I A104G E110P D123N L124M V138D | f-CTZ | 5.207 |
| K66N P84L E102A S103I A104G E110P D123N L124M V138D | CTZ | 0.513 |
| K66N P84L E102A S103I A104G E110P D123N L124M V138D | ZS2 | 0.041 |
| K66N P84L E102K E110P D123N | ZS2 | 0.112 |
| K66N P84L E102K E110P D123N | CTZ | 0.172 |
| K66R E102D E110P V138D | ZS2 | 1.560 |
| K66R E102D E110P V138D | CTZ | 0.674 |
| K66R E102K E110G D123N V138D | ZS2 | 1.611 |
| K66R E102K E110G D123N V138D | CTZ | 1.000 |
| K66R H79K P84K E102D S103I A104G L124M V138E | f-CTZ | 0.857 |
| K66R H79K P84K E102D S103I A104G L124M V138E | ZS2 | 0.459 |
| K66R H79K P84K E102D S103I A104G L124M V138E | CTZ | 0.407 |
| K66R P84A E102A S103I A104G E110A | ZS2 | 1.209 |
| K66R P84A E102A S103I A104G E110A | CTZ | 0.122 |
| K66R P84A E102K A104G E110A L124M V138E | f-CTZ | 10.315 |
| K66R P84A E102K A104G E110A L124M V138E | CTZ | 1.073 |
| K66R P84A E102K A104G E110A L124M V138E | ZS2 | 2.637 |
| K66R P84A E102S S103I A104G E110A L124M V138E | ZS2 | 0.587 |
| K66R P84A E102S S103I A104G E110A L124M V138E | CTZ | 1.302 |
| K66R P84L E102D S103T E110A V138E | ZS2 | 3.079 |
| K66R P84L E102D S103T E110A V138E | CTZ | 0.626 |
| N27D V29E A30G V31I A32V S33K A36I T37D L40T | ZS2 | 1.379 |
| N27D V29E A30G V31I A32V S33K A36I T37D L40T | CTZ | 0.779 |
| N27D V29S V31L A32V S33G A36N T37D L40R | ZS2 | 0.146 |
| N27D V29S V31L A32V S33G A36N T37D L40R | CTZ | 0.981 |
| N27D V29Y A30G V31L A32V S33K A36D L40V | f-CTZ | 0.155 |
| N27D V29Y A30G V31L A32V S33K A36D L40V | CTZ | 0.146 |
| N27D V29Y A30G V31L A32V S33K A36D L40V | ZS2 | 0.120 |
| N27Y V29G A30D V31L A32V S33V A36G T37D L40F Y97* | f-CTZ | 0.009 |
| N27Y V29G A30D V31L A32V S33V A36G T37D L40F Y97* | CTZ | 0.000 |
| N27Y V29G A30D V31L A32V S33V A36G T37D L40F Y97* | ZS2 | 0.001 |
| P53L | f-CTZ | 0.010 |
| P53L | CTZ | 0.007 |
| P53L | ZS2 | 0.002 |
| P84A E102A S103I D123L V138D | f-CTZ | 0.991 |
| P84A E102A S103I D123L V138D | CTZ | 0.473 |
| P84A E102A S103I D123L V138D | ZS2 | 0.825 |
| P84A E102A S103I E110P D123N | f-CTZ | 1.802 |
| P84A E102A S103I E110P D123N | CTZ | 1.343 |
| P84A E102A S103I E110P D123N | ZS2 | 1.409 |
| P84A E102A S103T E110A V138D | ZS2 | 4.252 |
| P84A E102A S103T E110A V138D | CTZ | 0.174 |
| P84A E102S S103T A104G E110A L124M V138E | ZS2 | 4.457 |
| P84A E102S S103T A104G E110A L124M V138E | CTZ | 0.375 |
| P84K E102A | f-CTZ | 0.009 |
| P84K E102A | CTZ | 0.000 |
| P84K E102A | ZS2 | 0.001 |
| P84K E102K S103I E110P L124M V138D | f-CTZ | 3.010 |
| P84K E102K S103I E110P L124M V138D | CTZ | 0.036 |
| P84K E102K S103I E110P L124M V138D | ZS2 | 0.536 |
| Q152H Q163D S170N G174S Q175E D177S A182Y | CTZ | 1.007 |
| Q152H Q163D S170N G174S Q175E D177S A182Y | f-CTZ | 0.404 |
| Q152H Q163D S170N G174S Q175E D177S A182Y | ZS2 | 0.347 |
| Q152H Q163D T167G S170V G174K Q175E K178N | CTZ | 1.749 |
| Q152H Q163D T167G S170V G174K Q175E K178N | f-CTZ | 0.215 |
| Q152H Q163D T167G S170V G174K Q175E K178N | ZS2 | 0.862 |
| Q152H Q163D T167S S170D G174K Q175E K178S A182Y G183N | f-CTZ | 0.509 |
| Q152H Q163D T167S S170D G174K Q175E K178S A182Y G183N | CTZ | 0.383 |
| Q152H Q163D T167S S170D G174K Q175E K178S A182Y G183N | ZS2 | 0.068 |
| Q152H Q163D T167S S170N A182L G183A | ZS2 | 0.698 |
| Q152H Q163D T167S S170N A182L G183A | CTZ | 1.197 |
| Q152H Q163D T167S S170N G174K A182L G183N | f-CTZ | 0.234 |
| Q152H Q163D T167S S170N G174K A182L G183N | CTZ | 0.144 |
| Q152H Q163D T167S S170N G174K A182L G183N | ZS2 | 0.159 |
| Q152H Q163S T167G S170V G174N G183A | ZS2 | 0.064 |
| Q152H Q163S T167G S170V G174N G183A | CTZ | 0.006 |
| Q152H Q163T T167G S170D G174N A182M G183A | f-CTZ | 0.478 |
| Q152H Q163T T167G S170D G174N A182M G183A | CTZ | 0.080 |
| Q152H Q163T T167G S170D G174N A182M G183A | ZS2 | 0.001 |
| Q152H Q163T T167G S170N Q175E A182L G183A | f-CTZ | 0.172 |
| Q152H Q163T T167G S170N Q175E A182L G183A | CTZ | 0.177 |
| Q152H Q163T T167G S170N Q175E A182L G183A | ZS2 | 0.087 |
| Q152H Q163T T167S S170D G174K K178N A182Y G183N | ZS2 | 0.089 |
| Q152H Q163T T167S S170D G174K K178N A182Y G183N | CTZ | 0.079 |
| Q152H S170D G174S K178T A182L | CTZ | 4.938 |
| Q152H S170D G174S K178T A182L | f-CTZ | 2.069 |
| Q152H S170D G174S K178T A182L | ZS2 | 2.672 |
| Q152H S170D Q175E A182Y | f-CTZ | 0.583 |
| Q152H S170D Q175E A182Y | CTZ | 0.615 |
| Q152H S170D Q175E A182Y | ZS2 | 0.723 |
| Q152K Q163D S170N G174K Q175E K178T A182L G183A | ZS2 | 1.183 |
| Q152K Q163D S170N G174K Q175E K178T A182L G183A | CTZ | 1.079 |
| Q152K Q163S T167G S170V G174K Q175E K180 | f-CTZ | 0.512 |
| Q152K Q163S T167G S170V G174K Q175E K180 | CTZ | 0.011 |
| Q152K Q163S T167G S170V G174K Q175E K180 | ZS2 | 0.136 |
| Q152K Q163S T167S S170N G174N K178S A182Y G183A | f-CTZ | 1.175 |
| Q152K Q163S T167S S170N G174N K178S A182Y G183A | CTZ | 0.906 |
| Q152K Q163S T167S S170N G174N K178S A182Y G183A | ZS2 | 0.819 |
| Q152K Q163T S170N G174N K178S A182Y G183A | f-CTZ | 0.299 |
| Q152K Q163T S170N G174N K178S A182Y G183A | CTZ | 0.194 |
| Q152K Q163T S170N G174N K178S A182Y G183A | ZS2 | 0.026 |
| Q152R Q163S T167G S170D G174K A182Y G183A | ZS2 | 1.402 |
| Q152R Q163S T167G S170D G174K A182Y G183A | CTZ | 1.050 |
| Q152R Q163S T167G S170T G174K K178S A182M G183A | f-CTZ | 1.864 |
| Q152R Q163S T167G S170T G174K K178S A182M G183A | CTZ | 1.171 |
| Q152R Q163S T167G S170T G174K K178S A182M G183A | ZS2 | 2.367 |
| Q152R Q163S T167G S170V G174K Q175E K178S A182Y G183A | ZS2 | 1.011 |
| Q152R Q163S T167G S170V G174K Q175E K178S A182Y G183A | CTZ | 0.992 |
| Q152R Q163S T167S S170D G174K Q175E K178N A182M | CTZ | 4.609 |
| Q152R Q163S T167S S170D G174K Q175E K178N A182M | f-CTZ | 1.020 |
| Q152R Q163S T167S S170D G174K Q175E K178N A182M | ZS2 | 3.139 |
| Q152R Q163T T167S S170N G174K K178S A182Y G183A | ZS2 | 0.413 |
| Q152R Q163T T167S S170N G174K K178S A182Y G183A | CTZ | 1.030 |
| Q152R S154F Q163D S170T G174N K178T A182Y | ZS2 | 0.003 |
| Q152R S154F Q163D S170T G174N K178T A182Y | CTZ | 0.020 |
| Q152R T167G G174S Q175E K178N A182Y G183A | f-CTZ | 0.404 |
| Q152R T167G G174S Q175E K178N A182Y G183A | CTZ | 0.703 |
| Q152R T167G G174S Q175E K178N A182Y G183A | ZS2 | 0.481 |
| Q152R T167G S170N K178S A182L G183N | ZS2 | 0.737 |
| Q152R T167G S170N K178S A182L G183N | CTZ | 0.807 |
| Q163S T167S S170T G174N K178T | ZS2 | 2.603 |
| Q163S T167S S170T G174N K178T | CTZ | 0.706 |
| Q163T T167S S170V G174N K178N A182Y G183A | f-CTZ | 0.135 |
| Q163T T167S S170V G174N K178N A182Y G183A | CTZ | 0.170 |
| Q163T T167S S170V G174N K178N A182Y G183A | ZS2 | 0.138 |
| S103I A104G E110G L124M V138D | f-CTZ | 1.701 |
| S103I A104G E110G L124M V138D | CTZ | 1.165 |
| S103I A104G E110G L124M V138D | ZS2 | 1.337 |
| T167S S170D G174N Q175E K178N A182L | ZS2 | 0.890 |
| T167S S170D G174N Q175E K178N A182L | CTZ | 0.850 |
| T96P Q163D T167S G174N K178S A182Y G183A | ZS2 | 0.273 |
| T96P Q163D T167S G174N K178S A182Y G183A | CTZ | 0.007 |
| V29A A30D V31I A32V S33N L40A | ZS2 | 1.164 |
| V29A A30D V31I A32V S33N L40A | CTZ | 1.046 |
| V29L A30G A32V S33G A36N T37A L40S | ZS2 | 1.976 |
| V29L A30G A32V S33G A36N T37A L40S | CTZ | 0.764 |
| V29L T96P Q162K Q163H T167S G174N A182Y | ZS2 | 0.877 |
| V29L T96P Q162K Q163H T167S G174N A182Y | CTZ | 1.017 |
| V29S A30G V31I A32V S33G A36D T37A | ZS2 | 0.142 |
| V29S A30G V31I A32V S33G A36D T37A | CTZ | 0.000 |

### Example 7 Construction of plasmids for prokaryotic expression of a Gaussia luciferase combinatorial mutant library with advantageous mutants

Based on the advantageous mutants with outstanding activities, together with the mutant library L1 or/and L3, a combinatorial mutant library with combined mutations was constructed in this example. The construction of a combinatorial mutant library based on the advantageous mutant B6 and mutant library L1 was illustrated as an example herein. Specifically, the combinatorial mutant library in the present example includes mutation sites involved in the mutant B6 and mutation sites involved in the mutant library L 1. Specific implementation for the library construction was as follows: i) constructing an insert fragment "insert 1" including mutation sites involved in the mutant B6, ii) constructing an insert fragment "insert 2", part of whose sequence was overlapped with insert 1, including mutation sites involved in the mutant library L1, and iii) constructing a linearized vector for recombination of insert 1 and insert 2.

For constructing the insert fragment "insert 1" including mutation sites involved in the mutant B6, primer sequences used for PCR are shown in Table 17, a reaction system is shown in Table 18, and reaction conditions are shown in Table 19.

**Table 17**

| Name | Sequence (5'-3') |
|---|---|
| Upstream primer: Primer insert-F | agccaatgctaggnnkgctggctgcactaggggatgtc(SEQ ID NO: 14) |
| Downstream primer: Primer insert-R | gcttttaagcagagattacctagaattcctatcaggagccgtg(SEQ ID NO: 15) |

**Table 18**

| Components | Volume (µL) |
|---|---|
| 2x PCR buffer for KOD FX Neo | 25 |
| 2 mM dNTPs | 10 |
| 10 µM Primer insert-F | 1 |
| 10 µM Primer insert-R | 1 |
| B6 mutant DNA as template (10 ng/µL) | 1 |
| KOD FX Neo | 1 |
| PCR grade water | 11 |
| Total volume | 50 |

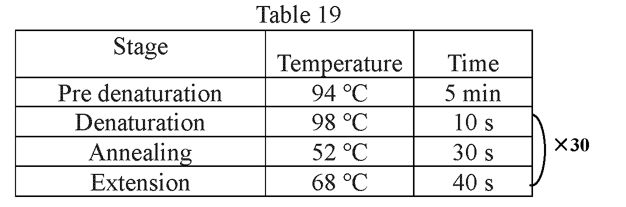

Dpnl enzyme, taken for 0.5 µL, was added into the reaction system and incubated at 37 °C for 3 hours to digest the template, followed by recovering products with a length of 464 bp from via gel as the insert fragment "insert 1".

For constructing the insert fragment "insert 2" including mutation sites involved in mutant library L1, primer sequences used for PCR are shown in Table 20, a reaction system is shown in Table 21, and reaction conditions are shown in Table 22.

**Table 20**

| Name | Sequence (5'-3') |
|---|---|
| Upstream primer: Primer insert-F | ccatgaatcacaaagtgcatatgaaaccaactgaaaacaatg(SEQ ID NO: 16) |
| Downstream primer: Primer insert-R | cctagtgcagccagcmnncctagcattggcttccatttct(SEQ ID NO: 17) |

**Table 21**

| Components | Volume (µL) |
|---|---|
| 2x PCR buffer for KOD FX Neo | 25 |
| 2 mM dNTPs | 10 |
| 10 µM Primer insert-F | 1 |
| 10 µM Primer insert-R | 1 |
| Mutant library L1 DNA as template (10 ng/µL) | 1 |
| KOD FX Neo | 1 |
| PCR grade water | 11 |
| Total volume | 50 |

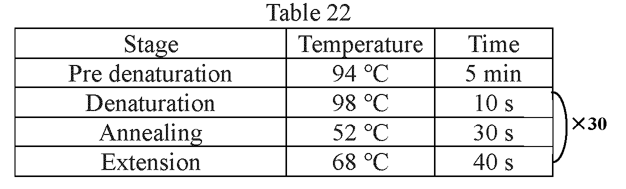

Dpnl enzyme, taken for 0.5 µL, was added to the reaction system and incubated at 37 °C for 3 hours to digest the template, followed by recovering products with a length of 185 bp via gel as the insert fragment "insert 2".

For constructing a linearized vector for recombination of insert 1 and insert 2, Taken pColdI vector as template, such a vector was linearized by PCR to facilitate recombination. With KOD FX neo enzyme, a PCR system was prepared and a PCR was conducted according to the instructions of the enzyme, where PCR primers are shown in Table 23, the reaction system of which is shown in Table 24, and reaction conditions are shown in Table 25.

**Table 23**

| Name | Sequence (5'-3') |
|---|---|
| Upstream primer: Primer vector-F | cacggctcctgataggaattctaggtaatctctgcttaaaagc(SEQ ID NO: 12) |
| Downstream primer: Primer vector-R | cattgttttcagttggtttcatatgcactttgtgattcatgg(SEQ ID NO: 13) |

**Table 24**

| Components | Volume (µL) |
|---|---|
| 2x KOD FX Neo PCR Buffer | 25 |
| 2 mM dNTPs | 10 |
| 10 µM Primer vector-F | 1.5 |
| 10 µM Primer vector-R | 1.5 |
| pColdI (10 ng/µL) | 2.5 |
| KOD FX Neo | 1 |
| PCR grade water | 8.5 |
| Total volume | 50 |

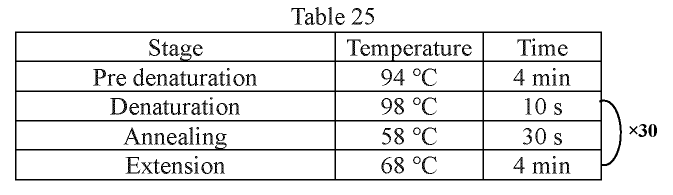

Dpnl enzyme, taken for 0.5 µL, was added into the reaction system and incubated at 37 °C for 3 hours to digest the template, followed by recovering products with a length of 4,278 bp via gel as the linearized vector. With Gibson Assembly kit, the insert 1, insert 2 and linearized vector were incubated at 50 °C for 30 minutes for recombination according to a reaction system as shown in Table 26.

**Table 26**

| Components | Volume (µL) |
|---|---|
| insert 1 (10 ng/µL) | 0.5 |
| insert 2 (10 ng/µL) | 0.5 |
| vector (10 ng/µL) | 0.5 |
| Gibson assembly (2 x) | 5 |
| PCR grade water | 3.5 |
| Total volume | 10 |

The above reaction products, taken for 2.5 µL, were transformed into DH5α competent cells, and coated on plate medium with ampicillin at a final ampicillin concentration of 100 µg/mL. Single colonies were picked from the plates the next day to extract plasmids. The obtained plasmids were the plasmids of the combinatorial mutant library including mutation sites of mutant B6 and library L1 both.

### Example 8 Substrate specificity test of Gaussia luciferase combinatorial mutants

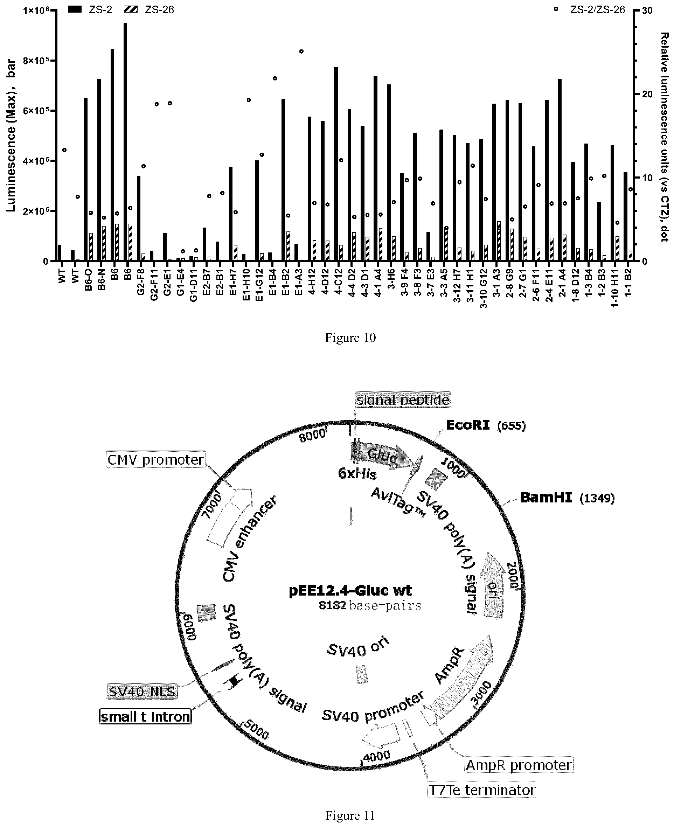

The new mutant library constructed in Example 7 was screened and tested for substrate specificity. Expression and purification schemes were the same as that in Example 5, and the activity assay scheme was the same as that in Example 6. The respective catalytic activities of combinatorial mutants on coelenterazine, fluoro-coelenterazine or coelenterazine derivative ZS2, and coelenterazine derivative ZS26 (purchased from BIOLAB) were assayed, and comparisons among the substrate specificities of the combinatorial mutants were based on the ratios of catalytic activities on two corresponding substrates. Figure 9 shows the comparison of catalytic activities on fluoro-coelenterazine relative to ZS26. The following four mutation combinations had significantly improved ratios of catalytic activities on fluoro-coelenterazine relative to ZS26, identifiers of which were: E1-A3 (F26R, V29F, A32V, S33E, A36V, L40I, K66P, H79K, P84L, E102S, S103T, A104G, E110P, L124M, V138E), G2-F11 (E24K, H79K, P84L, E102S, S103T, A104G, E110P, L124G, Q152R, Q163D, S170N, Q175E, K178T, A182M, G183N), G2-E1(H79K, P84L, E102S, S103T, A104G, E110P, L124I, Q152H, Q163D, T167S, S170T, G174K, A182M, G183A), and G2-F8 (H79K, P84L, E102S, S103T, A104G, E110P, L124M, Q152H, Q163D, T167S, S170T, G174K, A182M, G183A). Figure 10 shows the comparison of catalytic activities on ZS2 relative to ZS26. The following six mutation combinations had significantly improved ratios of catalytic activities on ZS2 relative to ZS26, and identifiers of which were: E1-A3 (F26R, V29F, A32V, S33E, A36V, L40I, K66P, H79K, P84L, E102S, S103T, A104G, E110P, L124M, V138E), E1-B4 (N27D, V29L, A30G, A32V, S33R, A36I, T37N, L40T, K66S, H79K, P84L, E102S, S103T, A104G, G109V, E110P, L124M, V138E), G2-E1 (H79K, P84L, E102S, S103T, A104G, E110P, L124I, Q152H, Q163D, T167S, S170T, G174K, A182M, G183A), G2-F11 (E24K, H79K, P84L, E102S, S103T, A104G, E110P, L124G, Q152R, Q163D, S170N, Q175E, K178T, A182M, G183N ), E1-G12 (F26L, V29L, A30D, V31I, S33K, A36I, T37E, K66I, H79K, P84L, E102S, S103T, A104G, E110P, L124M, V138E), and 4-C12(H79K, E102S, S103T, A104G, E110P, L124M, V138E).

The results of substrate specificity detection for Gaussia luciferase mutants are shown in Table 27.

**Table 27**

| No. | Mutation sites | measured value, CTZ | measured value, f-CTZ | measured value, ZS-2 | measured value, ZS-26 | ratio of catalytic activities, CTZ/ZS26 | ratio of catalytic activities, f-CTZ/ZS26 | ratio of catalytic activities, ZS2/ZS26 |
|---|---|---|---|---|---|---|---|---|
| E1-A3 | F26R, V29F, A32V, S33E,A36V, L40I, K66P,H79K, P84L, E102S, S103T, A104G, E110P, L124M, V138E | 328521 | 37761 | 69125 | 2755 | 119.24 | 13.71 | 25.09 |
| G2-F11 | E24K, H79K, P84L, E102S, S103T, A104G, E110P, L124G, Q152R, Q163D, S170N, Q175E, K178T, A182M, G183N | 165291 | 23049 | 40293 | 2145 | 77.058 | 10.75 | 18.78 |
| G2-E1 | H79K, P84L, E102S, S103T, A104G, E110P, L124I, Q152H, Q163D, T167S, S170T, G174K, A182M, G183A | 385814 | 62026 | 111198 | 5886 | 65.54 | 10.54 | 18.89 |
| G2-F8 | H79K, P84L, E102S, S103T, A104G, E110P, L124M, Q152H, Q163D, T167S, S170T, G174K, A182M, G183A | 569453 | 140085 | 339202 | 29855 | 19.07 | 4.69 | 11.36 |
| 3-7 E3 | V31Q, S33M, H79K, P84L, E102S, S103T, A104G, E110P, L124M, V138E | 197017 | 63522 | 116645 | 16876 | 11.67 | 3.76 | 6.91 |
| 3-8 F3 | H79K, P84L, E102S, S103T, A104G, E110S, L124M, V138E | 1566985 | 174157 | 511097 | 51706 | 30.30 | 3.37 | 9.88 |
| 3-9 F4 | H79K, P84L, E102S, S103T, A104G, E110L, L124M, V138E | 1041670 | 120346 | 349548 | 36035 | 28.90 | 3.34 | 9.70 |
| 3-11 H1 | H79K, P84L, E102S, S103T, A104G, E110T, L124M, V138E | 1414490 | 134686 | 470004 | 41156 | 34.39 | 3.27 | 11.42 |
| E1-B4 | N27D, V29L, A30G, A32V, S33R, A36I, T37N, L40T, K66S, H79K, P84L, E102S, S103T, A104G, G109V, E110P, L124M, V138E | 245213 | 5177 | 35088 | 1604 | 152.87 | 3.23 | 21.88 |
| E1-G12 | F26L, V29L, A30D, V31I, S33K, A36I, T37E, K66I, H79K, P84L, E102S, S103T, A104G, E110P, L124M, V138E | 689624 | 94900 | 401271 | 31521 | 21.87 | 3.01 | 12.73 |
| 1-3 B4 | H79R, E102S, S103T, A104G, E110P, L124M, V138E | 1198764 | 137462 | 468621 | 47313 | 25.33 | 2.91 | 9.90 |
| 3-10 G12 | H79K, P84L, E102S, S103T, A104G, E110R, L124M, V138E | 1559267 | 189329 | 486163 | 65527 | 23.79 | 2.89 | 7.42 |
| 3-12 H7 | H79K, P84L, E102S, S103T, A104G, E110K, L124M, V138E | 1504962 | 152368 | 503019 | 53289 | 28.24 | 2.86 | 9.44 |
| 4-3 D1 | N34K, H79K, P84L, E102S, S103T, A104G, E110P, L124M, V138E | 918168 | 270137 | 539314 | 97249 | 9.44 | 2.78 | 5.55 |
| 1-1 B2 | H79Q, E102S, S103T, A104G, E110P, L124M, V138E | 1590619 | 109898 | 353749 | 41163 | 38.64 | 2.67 | 8.59 |
| 1-2 B3 | H79T, E102S, S103T, A104G, E110P, L124M, V138E | 656156 | 61043 | 235613 | 23086 | 28.42 | 2.64 | 10.21 |
| 4-C12 | H79K, E102S, S103T, A104G, E110P, L124M, V138E | 1675889 | 167522 | 773694 | 64057 | 26.16 | 2.62 | 12.08 |
| 1-8 D12 | E102S, S103T, A104G, E110P, L124M, V138E | 811067 | 130801 | 393823 | 52275 | 15.51 | 2.50 | 7.53 |
| 4-4 D2 | S33V, N34D, H79K, P84L, E102S, S103T, A104G, E110P, L124M, V138E | 1253943 | 279682 | 606944 | 114796 | 10.92 | 2.44 | 5.29 |
| 4-H12 | E102S, S103T, A104G, E110R, L124M, V138E | 1324584 | 192739 | 576092 | 82832 | 15.99 | 2.33 | 6.95 |
| 3-3 A5 | V31E, H79K, P84L, E102S, S103T, A104G, E110P, L124M, V138E | 1177717 | 260083 | 523970 | 131853 | 8.93 | 1.97 | 3.97 |
| 4-D12 | E102S, S103T, A104G, E110R, L124M, V138E | 1159544 | 162648 | 559314 | 82532 | 14.04 | 1.97 | 6.78 |
| 2-7 G1 | V29L, H79K, P84L, E102S, S103T, A104G, E110P, L124M, V138E | 952907 | 186596 | 630265 | 96469 | 9.87 | 1.93 | 6.53 |
| 1-10 H11 | P84E, E102S, S103T, A104G, E110R, L124M, V138E | 811067 | 193268 | 462558 | 100262 | 8.08 | 1.93 | 4.61 |
| 3-1 A3 | V31T, H79K, P84L, E102S, S103T, A104G, E110P, L124M, V138E | 1378952 | 303636 | 627815 | 158559 | 8.69 | 1.91 | 3.96 |
| 2-1 A4 | N27S, H79K, P84L, E102S, S103T, A104G, E110P, L124M, V138E | 1247610 | 198926 | 725685 | 104808 | 11.90 | 1.90 | 6.92 |
| 2-6 F11 | V29F, H79K, P84L, E102S, | 707229 | 92917 | 456799 | 50016 | 14.14 | 1.86 | 9.13 |
| | S103T, A104G, E110P, L124M, V138E | | | | | | | |
| 2-4 E11 | V29E, H79K, P84L, E102S, S103T, A104G, E110P, L124M, V138E | 1105429 | 169250 | 641725 | 93197 | 11.86 | 1.82 | 6.89 |
| 2-8 G9 | V29D, H79K, P84L, E102S, S103T, A104G, E110P, L124M, V138E | 1331334 | 232963 | 642254 | 128566 | 10.35 | 1.81 | 5.00 |
| B6 | H79K, P84L, E102S, S103T, A104G, E110P, L124M, V138E | 1506076 | 264603.3 | 792935 | 137323.5 | 10.96 | 1.92 | 5.77 |
| WT | | 493363.5 | 10186.5 | 54832 | 5338 | 92.42 | 1.90 | 10.27 |

### Example 9 Design and construction of plasmids for eukaryotic expression of Gaussia luciferase

The expression plasmid pET28a-Gluc wt with the signal peptide was used as template DNA to construct a plasmid for eukaryotic expression of Gaussia luciferase, where primer sequences used for a PCR to prepare the insert fragment of the eukaryotic expression plasmid are shown in Table 28, a reaction system of which is shown in Table 29, and reaction conditions are shown in Table 30.

**Table 28**

| Name | Sequence (5'-3') |
|---|---|
| Upstream primer Primer insert-F | caccaccatcacggcagcaaaccaactgaaaacaatg(SEQ ID NO: 18) |
| Downstream primer Primer insert-R | gatatcattcagtccggagccatcaccaccggcacc(SEQ ID NO: 19) |

**Table 29**

| Components | Volume (µL) |
|---|---|
| 2x PCR buffer for KOD FX Neo | 25 |
| 2 mM dNTPs | 10 |
| 10 µM Primer insert-F | 1 |
| 10 µM Primer insert-R | 1 |
| template DNA (10 ng/µL) | 2 |
| KOD FX Neo | 1 |
| PCR grade water | 8.5 |
| Total volume | 50 |

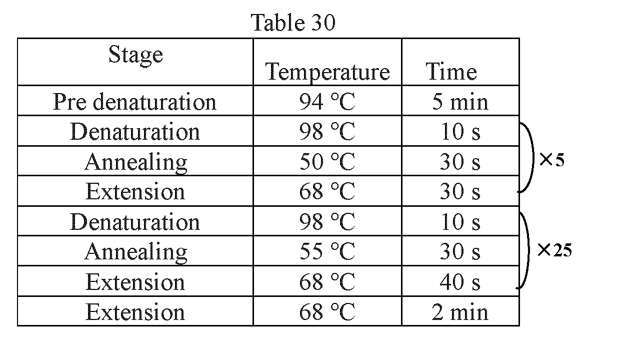

Dpnl enzyme, taken for 0.5 µL, was added into the reaction system and incubated at 37 °C for 3 hours to digest the template, followed by recovering products with a length of 618 bp via gel as the insert fragment.

With pEE12.4 vector with a histidine-tag for purification at its N-terminal and an Avitag for biotinylation at its C-terminal as template, such a vector was linearized by PCR to facilitate recombination with the insert fragment. With KOD FX neo enzyme, a PCR system was prepared and a PCR was conducted according to the instructions of the enzyme, PCR primers for which are shown in Table 31, the reaction system is shown in Table 32, and reaction conditions are shown in Table 33.

**Table 31**

| Name | Sequence (5'-3') |
|---|---|
| Upstream primers | ggtgccggtggtgatggctccggactgaatgatatc(SEQ ID NO: 20) |
| downstream primers | cattgttttcagttggtttgctgccgtgatggtggtg(SEQ ID NO: 21) |

**Table 32**

| Components | Volume (µL) |
|---|---|
| 2x KOD FX Neo PCR Buffer | 25 |
| 2 mM dNTPs | 10 |
| 10 µM Primer vector-F | 1.5 |
| 10 µM Primer vector-R | 1.5 |
| pEE12.5 (10 ng/µL) | 1 |
| KOD FX Neo | 1 |
| PCR grade water | 10 |
| Total volume | 50 |

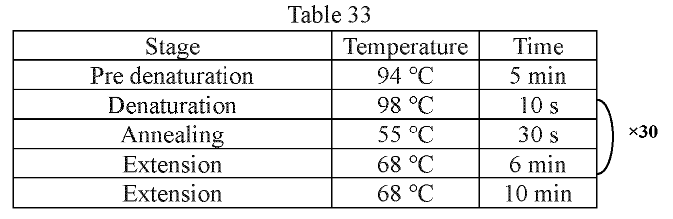

Dpnl enzyme, taken for 0.5 µL, was added into the reaction system and incubated at 37 °C for 3 hours to digest the template, followed by recovering products with a length of about 7,564 bp via gel as the linearized vectors. With In-Fusion Cloning kit (Takara) the insert fragment and the linearized vector obtained in this Example were incubated at 50 °C for 15 minutes for recombination, based on a reaction system shown in Table 34.

**Table 34**

| Components | Volume (µL) |
|---|---|
| insert (10 ng/µL) | 5 |
| vector (10 ng/µL) | 3 |
| Premix | 2 |
| Total volume | 10 |

The above reaction products, taken for 2.5 µL, were transformed into DH5α competent cells, and coated on plate medium with ampicillin at a final concentration of 100 µg/mL. Single colonies were picked from the plates the next day to extract plasmids therein for sequencing, ensuring the target fragment being correctly inserted into the vector and the obtained plasmids being the plasmids (pEE12.4-Gluc wt) encoding wild-type luciferase for eukaryotic expression. The obtained plasmids were verified, the specific structure of which is shown in Figure 11, in line with the experimental expectations. The plasmid constructing method of the mutants was the same as that of the wild type luciferase (pEE12.4-Gluc wt) for eukaryotic expression, and the template of the insert fragment was the corresponding mutant.

### Example 10 Eukaryotic expression and protein purification of advantageous mutants of Gaussia luciferase

The plasmids pEE12.4-Gluc wt and pEE12.4-Gluc B6 obtained in Example 9 were individually transfected into 30 mL of Expi-CHO cells according to ExpiFectamine^{™} CHO transfection kit (Gibco, A29129). After 7 days of the transfection, the cells with cell viability measured less than 90%, and were subject to centrifugation at 4 °C at 8,000 rpm for 10 minutes, to collect the supernatant.

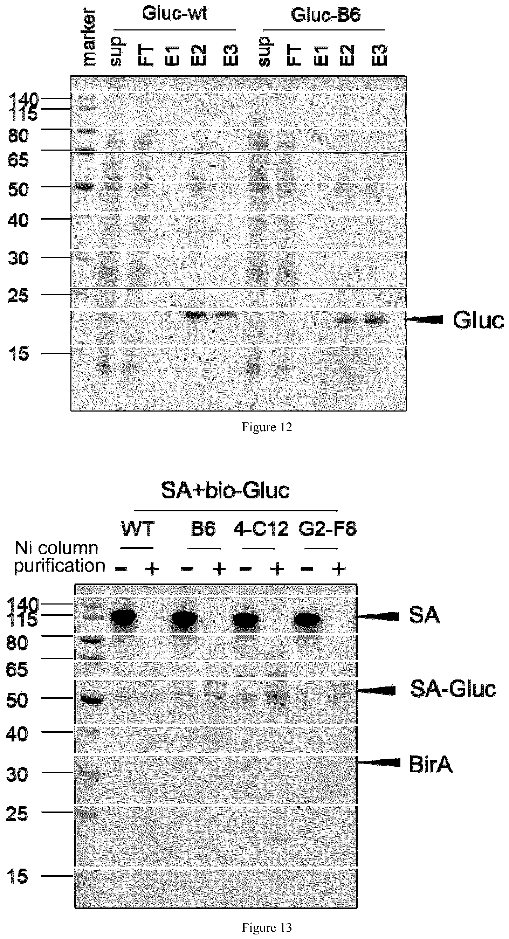

2 ML of HisTrap FF packing were loaded into a manual column (purchased from Sangon Biotech, #F506607-0001, affinity chromatography column, unloaded), and flushed and balanced with 30 mL of binding buffer. Then about 30 mL of the filtered cell supernatant were added to the column, washed for 10 times (10 mL/time) with washing solution (50 mM Tris, pH 8.0, 250 mM NaCl, 10 mM imidazole) and then eluted with 500 µL of eluent (50 mM Tris, pH 8.0, 250 mM NaCl, 300 mM imidazole) for 4-5 times. The eluted protein was collected and detected with 12% SDS-PAGE. The purification results are shown in Figure 12, which indicates that a relatively pure Glue protein was obtained from components of the elution 2 and elution 3.

### Example 11 Coupling and activity detection of advantageous mutants of Gaussia luciferase

The Gluc protein obtained in Example 10 includes an AviTag, that is, AviTag-Gluc, which could be biotinylated into Biotin-Avitag-Gluc by BirA enzyme (Avidity, BIRA500). A reaction system for the biotinylation is shown in Table 35.

**Table 35**

| Components | Volume (µL) |
|---|---|
| AviTag-Gluc (1 mg/mL) | 12 |
| BiomixA | 10 |
| BiomixB | 10 |
| BirA (1 mg/mL) | 3 |
| H2O | 65 |
| Total volume | 100 |

Protein SA-Gluc was prepared by adding protein SA into the system shown in Table 35, which was subject to further purification with his-tag of Glue, to obtain SA-Gluc with higher purity. The purification result is shown in Figure 13.

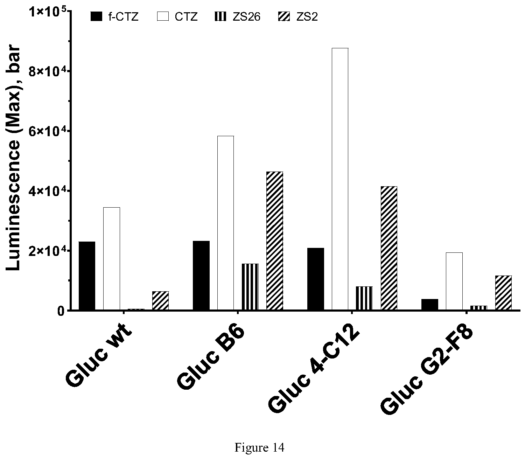

Activities of SA-Gluc (Glue wt) and its mutants SA-Gluc B6, SA-Gluc 4-C12 and SA-Gluc G2-F8 was assayed according to the method described in Example 6, and the results are shown in Figure 14.

Reference throughout this specification to "an embodiment," "some embodiments," "one embodiment", "another example," "an example," "a specific example," or "some examples," means that a particular feature, structure, material, or characteristic described in connection with the embodiment or example is included in at least one embodiment or example of the present disclosure. Thus, the appearances of the phrases such as "in some embodiments," "in one embodiment", "in an embodiment", "in another example," "in an example," "in a specific example," or "in some examples," in various places throughout this specification are not necessarily referring to the same embodiment or example of the present disclosure. Furthermore, the particular features, structures, materials, or characteristics may be combined in any suitable manner in one or more embodiments or examples. Besides, any different embodiments and examples and any different characteristics of embodiments and examples may be combined by those skilled in the art without contradiction.

Although explanatory embodiments have been shown and described, it would be appreciated by those skilled in the art that the above embodiments cannot be construed to limit the present disclosure, and changes, alternatives, and modifications can be made in the embodiments in the scope of the present disclosure.

## Claims

1. A mutant, comprising at least one mutation, compared with an amino acid sequence as shown in SEQ ID NO: 3, occurred at a position(s) selected from positions 24, 26, 27, 29, 30, 31, 32, 33, 36, 37, 40, 66, 79, 84, 88, 102, 103, 104, 110, 123, 124, 138, 152, 163, 167, 170, 174, 175, 178, 182, and 183 thereof.

2. The mutant according to claim 1, comprising at least one of the following mutations compared with the amino acid sequence as shown in SEQ ID NO: 3:
1) E at position 24 is mutated into K;
2) F at position 26 is mutated into R or L;
3) N at position 27 is mutated into D;
4) V at position 29 is mutated into F or L;
5) A at position 30 is mutated into G or D;
6) V at position 31 is mutated into I;
7) A at position 32 is mutated into V;
8) S at position 33 is mutated into E, R or K;
9) A at position 36 is mutated into V or I;
10) T at position 37 is mutated into N or E;
11) L at position 40 is mutated into I or T;
12) K at position 66 is mutated into P, S, I, R or N;
13) H at position 79 is mutated into K;
14) P at position 84 is mutated into A, L, K or V;
15) K at position 88 is mutated into R;
16) E at position 102 is mutated into D, A, S, K or N;
17) S at position 103 is mutated into T;
18) A at position 104 is mutated into G;
19) E at position 110 is mutated into P, G or A;
20) D at position 123 is mutated into N;
21) L at position 124 is mutated into M, G or I;
22) V at position 138 is mutated into E or D;
23) Q at position 152 is mutated into R or H;
24) Q at position 163 is mutated into D;
25) S at position 170 is mutated into N or T;
26) G at position 174 is mutated into K;
27) Q at position 175 is mutated into E;
28) K at position 178 is mutated into T;
29) A at position 182 is mutated into M; and
30) G at position 183 is mutated into N or A.

3. The mutant according to claim 1, comprising the following mutations compared with the amino acid sequence as shown in SEQ ID NO: 3, occurred at positions:
1) 79, 84, 102, 103, 104, 124 and 138; or
2) 66, 79, 84, 103, 104 and 138; or
3) 66, 79, 84, 102, 103, 104, 124 and 138; or
4) 79, 84, 102, 103, 104, 124 and 138; or
5) 66, 79, 102, 103, 104 and 138; or
6) 66, 79, 84, 102, 104, 124 and 138; or
7) 79, 84, 102, 104, 110 and 124; or
8) 84, 102, 104, 110 and 124; or
9) 66, 79, 84, 102, 103, 104, 110, 124 and 138; or
10) 66, 84, 102, 103, 104, 110, 124 and 138; or
11) 66, 84, 88, 102, 103, 104 and 124; or
12) 66, 84, 103, 104, 110, 123, 124 and 138; or
13) 84, 102, 103, 104 and 110; or
14) 66, 79, 84, 102, 103, 104 and 138; or
15) 66, 84, 102, 103, 104, 110, 124 and 138; or
16) 26, 29, 32, 33, 36, 40, 66, 79, 84, 102, 103, 104, 110, 124 and 138; or
17) 24, 79, 84, 102, 103, 104, 110, 124, 152, 163, 170, 175, 178, 182 and 183; or
18) 79, 84, 102, 103, 104, 110, 124, 152, 163, 167, 170, 174, 182 and 183; or
19) 79, 84, 102, 103, 104, 110, 124, 152, 163, 167, 170, 174, 182 and 183; or
20) 27, 29, 30, 32, 33, 36, 37, 40, 66, 79, 84, 102, 103, 104, 109, 110, 124 and 138; or
21) 26, 29, 30, 31, 33, 36, 37, 66, 79, 84, 102, 103, 104, 110, 124 and 138; or
22) 79, 102, 103, 104, 110, 124 and 138.

4. The mutant according to any one of claims 1 to 3, comprising the following mutations, compared with the amino acid sequence as shown in SEQ ID NO: 3:
1) H at position 79 is mutated into K, P at position 84 is mutated into A, E at position 102 is mutated into D, S at position 103 is mutated into T, A at position 104 is mutated into G, L at position 124 is mutated into M, and V at position 138 is mutated into E; or
2) K at position 66 is mutated into R, H at position 79 is mutated into K, P at position 84 is mutated into L, S at position 103 is mutated into T, A at position 104 is mutated into G, and V at position 138 is mutated into E; or
3) K at position 66 is mutated into R, H at position 79 is mutated into K, P at position 84 is mutated into A, E at position 102 is mutated into A, S at position 103 is mutated into T, A at position 104 is mutated into G, L at position 124 is mutated into M, and V at position 138 is mutated into D; or
4) H at position 79 is mutated into K, P at position 84 is mutated into L, E at position 102 is mutated into S, S at position 103 is mutated into T, A at position 104 is mutated into G, L at position 124 is mutated into M, and V at position 138 is mutated into E; or
5) K at position 66 is mutated into N, H at position 79 is mutated into K, E at position 102 is mutated into A, S at position 103 is mutated into T, A at position 104 is mutated into G, and V at position 138 is mutated into E; or
6) K at position 66 is mutated into R, H at position 79 is mutated into K, P at position 84 is mutated into A, E at position 102 is mutated into S, A at position 104 is mutated into G, L at position 124 is mutated into M, and V at position 138 is mutated into E; or
7) H at position 79 is mutated into K, P at position 84 is mutated into A, E at position 102 is mutated into D, A at position 104 is mutated into G, E at position 110 is mutated into A, and L at position 124 is mutated into M; or
8) P at position 84 is mutated into A, E at position 102 is mutated into K, A at position 104 is mutated into G, E at position 110 is mutated into A, and L at position 124 is mutated into M; or
9) K at position 66 is mutated into N, H at position 79 is mutated into K, P at position 84 is mutated into L, E at position 102 is mutated into S, S at position 103 is mutated into T, A at position 104 is mutated into G, E at position 110 is mutated into P, L at position 124 is mutated into M, and V at position 138 is mutated into D; or
10) K at position 66 is mutated into R, P at position 84 is mutated into A, E at position 102 is mutated into A, S at position 103 is mutated into T, A at position 104 is mutated into G, E at position 110 is mutated into P, L at position 124 is mutated into M, and V at position 138 is mutated into E; or
11) K at position 66 is mutated into R, P at position 84 is mutated into L, K at position 88 is mutated into R, E at position 102 is mutated into A, S at position 103 is mutated into T, A at position 104 is mutated into G, and L at position 124 is mutated into M; or
12) K at position 66 is mutated into N, P at position 84 is mutated into K, S at position 103 is mutated into T, A at position 104 is mutated into G, E at position 110 is mutated into G, D at position 123 is mutated into N, L at position 124 is mutated into M, and V at position 138 is mutated into E; or
13) P at position 84 is mutated into K, E at position 102 is mutated into A, S at position 103 is mutated into T, A at position 104 is mutated into G, and V at position 138 is mutated into D; or
14) K at position 66 is mutated into N, H at position 79 is mutated into K, P at position 84 is mutated into A, E at position 102 is mutated into D, S at position 103 is mutated into T, A at position 104 is mutated into G, and V at position 138 is mutated into D; or
15) K at position 66 is mutated into N, P at position 84 is mutated into V, E at position 102 is mutated into N, S at position 103 is mutated into T, A at position 104 is mutated into G, E at position 110 is mutated into A, L at position 124 is mutated into M, and V at position 138 is mutated into D; or
16) F at position 26 is mutated into R, V at position 29 is mutated into F, A at position 32 is mutated into V, S at position 33 is mutated into E, A at position 36 is mutated into V, L at position 40 is mutated into I, K at position 66 is mutated into P, H at position 79 is mutated into K, P at position 84 is mutated into L, E at position 102 is mutated into S, S at position 103 is mutated into T, A at position 104 is mutated into G, E at position 110 is mutated into P L at position 124 is mutated into M and V at position 138 is mutated into E; or
17) E at position 24 is mutated into K, H at position 79 is mutated into K, P at position 84 is mutated into L, E at position 102 is mutated into S, S at position 103 is mutated into T, A at position 104 is mutated into G, E at position 110 is mutated into P, L at position 124 is mutated into G, Q at position 152 is mutated into R, Q at position 163 is mutated into D, S at position 170 is mutated into N, Q at position 175 is mutated into E, K at position 178 is mutated into T, A at position 182 is mutated into M and G at position 183 is mutated into N; or
18) H at position 79 is mutated into K, P at position 84 is mutated into L, E at position 102 is mutated into S, S at position 103 is mutated into T, A at position 104 is mutated into G, E at position 110 is mutated into P, L at position 124 is mutated into I, Q at position 152 is mutated into H, Q at position 163 is mutated into D, T at position 167 is mutated into S, S at position 170 is mutated into T, G at position 174 is mutated into K, A at position 182 is mutated into M and G at position 183 is mutated into A; or
19) H at position 79 is mutated into K, P at position 84 is mutated into L, E at position 102 is mutated into S, S at position 103 is mutated into T, A at position 104 is mutated into G, E at position 110 is mutated into P, L at position 124 is mutated into M, Q at position 152 is mutated into H, Q at position 163 is mutated into D, T at position 167 is mutated into S, S at position 170 is mutated into T, G at position 174 is mutated into K, A at position 182 is mutated into M and G at position 183 is mutated into A; or
20) N at position 27 is mutated into D, V at position 29 is mutated into L, A at position 30 is mutated into G, A at position 32 is mutated into V, S at position 33 is mutated into R, A at position 36 is mutated into L, T at position 37 is mutated into N, L at position 40 is mutated into T, K at position 66 is mutated into S, H at position 79 is mutated into K, P at position 84 is mutated into L, E at position 102 is mutated into S, S at position 103 is mutated into T A at 104 is mutated into G, G at 109 is mutated into V, E at 110 is mutated into P, L at 124 is mutated into M, and V at 138 is mutated into E; or
21) F at position 26 is mutated into L, V at position 29 is mutated into L, A at position 30 is mutated into D, V at position 31 is mutated into I, S at position 33 is mutated into K, A at position 36 is mutated into I, T at position 37 is mutated into E, K at position 66 is mutated into I, H at position 79 is mutated into K, P at position 84 is mutated into L, E at position 102 is mutated into S, S at position 103 is mutated into T, A at position 104 is mutated into G E at position 110 is mutated into P, L at position 124 is mutated into M, and V at position 138 is mutated into E; or
22) H at position 79 is mutated into K, E at position 102 is mutated into S, S at position 103 is mutated into T, A at position 104 is mutated into G, E at position 110 is mutated into P, L at position 124 is mutated into M, and V at position 138 is mutated into E.

5. A nucleic acid molecule, encoding a mutant of any one of claims 1 to 4,
optionally, wherein the nucleic acid molecule comprises a nucleotide sequence as shown in one of SEQ ID NOs: 22-43.

6. An expression vector, comprising a nucleic acid molecule of claim 5.

7. A recombinant cell, comprising a nucleic acid molecule of claim 5, an expression vector of claim 6, or a mutant of any one of claims 1 to 4,
optionally, the recombinant cell being *Escherichia coli*, *Saccharomyces cerevisiae*, an insect cell or a mammalian cell.

8. A method for detecting a nucleic acid, comprising:
i) exposing an expression vector to an environment suitable for protein expression, wherein the expression vector comprises the nucleic acid to be detected and a nucleic acid molecule of claim 5, the nucleic acid to be detected is operably connected to and expressed together with the nucleic acid molecule of claim 5;
ii) introducing a substrate for Gaussia luciferase or analog thereof into the environment suitable for protein expression; and
iii) determining an expression of the nucleic acid to be detected based on a fluorescence intensity change in the environment suitable for protein expression.

9. A method of preparing a mutant, comprising:
i) constructing an expression vector of claim 6; and
ii) introducing the expression vector into a host cell to obtain the mutant.

10. Use of a mutant of any one of claims 1 to 4 in the preparation of a kit for detecting a content of analyte, wherein a specific recognition protein of the analyte allows to form a complex with the mutant,
optionally, the kit further comprises a substrate for Gaussia luciferase,
optionally, the use comprises determining the content of the analyte based on a fluorescence intensity change caused by the mutant before and after introducing the substrate for Gaussia luciferase or analog thereof,
optionally, the kit further comprises the specific recognition protein of the analyte,
optionally, the substrate comprises coelenterazine, fluoro-coelenterazine or a coelenterazine derivative,
optionally, the coelenterazine derivative comprises a coelenterazine derivative ZS2 or a coelenterazine derivative ZS26.

11. A kit for detecting a content of analyte, comprising a mutant of any one of claims 1 to 4, wherein a specific recognition protein of the analyte allows to form a complex with the mutant.

12. The kit of claim 11, further comprising a substrate for Gaussia luciferase,
optionally, the substrate comprises coelenterazine, fluoro or coelenterazine or a coelenterazine derivative,
optionally, the coelenterazine derivative comprises a coelenterazine derivative ZS2 or a coelenterazine derivative ZS26.

13. The kit of claim 11, further comprising the specific recognition protein of the analyte.

14. A method for detecting a content of analyte, comprising:
i) rendering a specific recognition protein of the analyte and a mutant of any one of claims 1 to 4 to form a complex,
ii) contacting the analyte with the complex,
iii) introducing a substrate for Gaussia luciferase or analog thereof into the system, and
iv) determining the content of the analyte based on a fluorescence intensity change caused by the mutant before and after introducing the substrate for Gaussia luciferase or analog thereof,
optionally, the substrate for Gaussia luciferase comprises coelenterazine, fluo-coelenterazine, or a coelenterazine derivative,
optionally, the coelenterazine derivative comprises a coelenterazine derivative ZS2 or a coelenterazine derivative ZS26.

15. A method of screening a substrate for Gaussia luciferase, comprising:
i) contacting a mutant of any one of claims 1 to 4 with the substrate to be screened, and
ii) determining whether the substrate to be screened as a target substrate or not based on whether a mixture in step i) emitting a chemical light signal or not.

16. The method of claim 15, wherein the mixture in step i) emitting a chemical light signal indicates that the substrate to be screened is the target substrate.
